(19) Europäisches Patentamt
European Patent Office
Office européen des brevets

(11) EP 4 036 080 A1

(12) EUROPEAN PATENT APPLICATION

(43) Date of publication:
**03.08.2022 Bulletin 2022/31**

(21) Application number: **21154618.9**

(22) Date of filing: **01.02.2021**

(51) International Patent Classification (IPC):
***C07D 213/61*** *(2006.01)* ***C07D 213/84*** *(2006.01)*
***C07C 255/35*** *(2006.01)* ***C07C 255/51*** *(2006.01)*
***H01L 51/00*** *(2006.01)*

(52) Cooperative Patent Classification (CPC):
**C07D 213/61; C07C 255/35; C07C 255/51; C07D 213/84; H01L 51/0051; H01L 51/0067;** H01L 51/5088

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Novaled GmbH**
**01099 Dresden (DE)**

(72) Inventors:
- **SCHULZE, Benjamin**
  **01099 Dresden (DE)**
- **NÜLLEN, Max Peter**
  **01099 Dresden (DE)**
- **WUDARCZYK, Jakob**
  **01099 Dresden (DE)**
- **ROSENOW, Thomas**
  **01099 Dresden (DE)**
- **LANGGUTH, Oliver**
  **01099 Dresden (DE)**
- **LUSCHTINETZ, Regina**
  **01099 Dresden (DE)**

(74) Representative: **Michalski Hüttermann & Partner Patentanwälte mbB**
**Kaistraße 16A**
**40221 Düsseldorf (DE)**

(54) **ORGANIC COMPOUND OF FORMULA (I) FOR USE IN ORGANIC ELECTRONIC DEVICES, AN ORGANIC ELECTRONIC DEVICE COMPRISING A COMPOUND OF FORMULA (I) AND A DISPLAY DEVICE COMPRISING THE ORGANIC ELECTRONIC DEVICE**

(57) The present invention relates to a compound of formula (I) and an organic electronic device comprising a semiconductor layer which comprises a compound of formula (I).

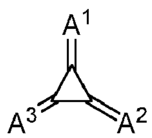

(I)

wherein $A^1$, $A^2$ and $A^3$ are represented by formula (II), (III), (IV), respectively

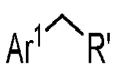 (II), 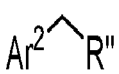 (III), and 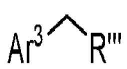 (IV)

EP 4 036 080 A1

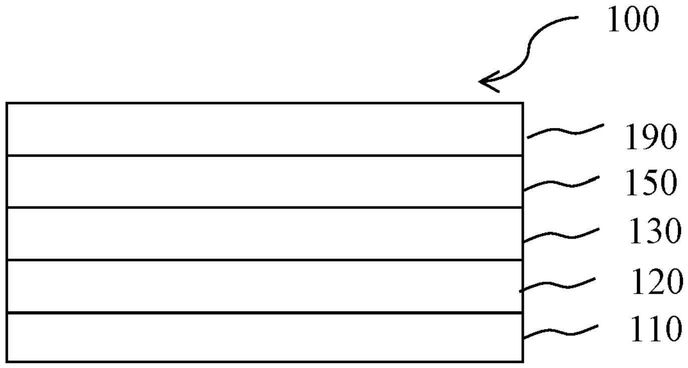


FIG. 1

## Description

### Technical Field

The present invention relates to an organic compound of formula (I) for use in organic electronic devices, an organic electronic device comprising a compound of formula (I) and a display device comprising the organic electronic device

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.
When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.
Performance of an organic light emitting diode may be affected by characteristics of the semiconductor layer, and among them, may be affected by characteristics of metal complexes which are also contained in the semiconductor layer.
There remains a need to improve performance of organic semiconductor materials, semiconductor layers, as well as organic electronic devices thereof, in particular to achieve improved performance of organic electronic devices in particular improved operating voltage, and/or current efficiency and/or stability in particular improved lifetime and/or improved operating voltage stability over time through improving the characteristics of the compounds comprised therein.
Additionally, there is a need to provide compounds with improved thermal properties, in particular decreased volatility and glass transition temperature allowing access of organic electronic devices with improved performance, and/or stability.
In the European patent application EP2180029A1, the use of radialenes are disclosed. The device according to the invention has been compared to said application.

### DISCLOSURE

An aspect of the present invention provides an organic compound for use in organic electronic devices of formula (I):

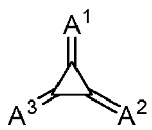

(I)

wherein $A^1$, $A^2$ and $A^3$ are represented by formula (II), (III), (IV), respectively

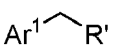 (II), 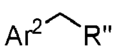 (III), and 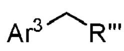 (IV)

whereby $Ar^1$, $Ar^2$ and $Ar^3$ are independently selected from substituted or unsubstituted aryl, substituted or unsubstituted $C_6$ to $C_{24}$ aryl, substituted or unsubstituted heteroaryl or $C_2$ to $C_{24}$ heteroaryl, wherein the substituents on $Ar^1$, $Ar^2$ and $Ar^3$ are independently selected form an electron-withdrawing group, $NO_2$, CN, halogen, Cl, F, partially fluorinated or perfluorinated alkyl and partially fluorinated or perfluorinated $C_1$ to $C_{12}$ alkyl, partially fluorinated or perfluorinated alkoxy, partially fluorinated or perfluorinated $C_1$ to $C_6$ alkoxy or D;
R', R" and R''' are independently selected from substituted or unsubstituted aryl, substituted or unsubstituted $C_6$ to $C_{18}$ aryl, substituted or unsubstituted heteroaryl, substituted or unsubstituted $C_2$ to $C_{18}$ heteroaryl, electron-with-

drawing group, partially fluorinated or perfluorinated alkyl, partially fluorinated or perfluorinated $C_1$ to $C_6$ alkyl, halogen, F, CN, and $NO_2$;
wherein the compound of formula (I) contains at least one $NO_2$ group;

or a salt of a radical anion of formula (I).

It should be noted that throughout the application and the claims any $A^n$, $R^n$ etc. always refer to the same moieties, unless otherwise noted.

In the present specification, when a definition is not otherwise provided, "partially fluorinated" refers to a $C_1$ to $C_8$ alkyl group in which only part of the hydrogen atoms are replaced by fluorine atoms.

In the present specification, when a definition is not otherwise provided, "perfluorinated" refers to a $C_1$ to $C_8$ alkyl group in which all hydrogen atoms are replaced by fluorine atoms.

In the present specification, when a definition is not otherwise provided, "substituted" refers to one substituted with a deuterium, $C_1$ to $C_{12}$ alkyl and $C_1$ to $C_{12}$ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a $C_1$ to $C_{12}$ alkyl group. More specifically, the alkyl group may be a $C_1$ to $C_{10}$ alkyl group, a $C_1$ to $C_8$ alkyl group, a $C_1$ to $C_6$ alkyl group, or a $C_1$ to $C_4$ alkyl group. For example, a $C_1$ to $C_4$ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a sec-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphtyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common $sp^2$-hybridized carbon atoms

The term "$NO_2$" represents - $NO_2$, i.e. the $NO_2$ is bonded by its nitrogen moiety.

The term "salt" refers to metal, ammonium, or radical cation salt of the radical anion of formula (I) preferably a radical cation salt.

The term "electron-withdrawing group" refers to a chemical group in a molecule, which can draw electrons away from an adjacent part of the molecule. The distance over which the electron-withdrawing group can exert its effect, namely the number of bonds over which the electron-withdrawing effect spans, is extended by conjugated pi-electron systems such as aromatic systems. Examples of electron-withdrawing groups include $NO_2$, CN, halogen, Cl, F, partially fluorinated or perfluorinated alkyl and partially fluorinated or perfluorinated $C_1$ to $C_{12}$ alkyl, partially fluorinated or perfluorinated alkoxy, partially fluorinated or perfluorinated $C_1$ to $C_6$ alkoxy.

In the present specification, the single bond refers to a direct bond.

The term "free of', "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is

in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms "anode", "anode layer" and "anode electrode" are used synonymously.

The terms "cathode", "cathode layer" and "cathode electrode" are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

**Advantageous Effects**

Surprisingly, it was found that the organic compound of the present invention solves the problem underlying the present invention by enabling devices in various aspects superior over the organic electroluminescent devices known in the art, in particular with respect to operating voltage over lifetime.

Preferably the compound of the present invention is a compound of formula (I).

According to one embodiment of the present invention, $Ar^1$ is substituted with at least one $NO_2$ group.

According to one embodiment of the present invention, the substituent on $Ar^1$, $Ar^2$ and $Ar^3$ are independently selected from $NO_2$, CN, halogen, Cl, F, partially fluorinated or perfluorinated alkyl and partially fluorinated or perfluorinated $C_1$ to $C_{12}$ alkyl, partially fluorinated or perfluorinated alkoxy, partially fluorinated or perfluorinated $C_1$ to $C_6$ alkoxy, preferably $NO_2$, CN, halogen, Cl, F, and partially fluorinated or perfluorinated $C_1$ to $C_{12}$ or D, more preferably $NO_2$, CN, F, partially fluorinated or perfluorinated alkyl and partially fluorinated or perfluorinated $C_1$ to $C_{12}$ alkyl, partially fluorinated or perfluorinated alkoxy, partially fluorinated or perfluorinated $C_1$ to $C_6$ alkoxy or D, more preferably $NO_2$, CN, F, perfluorinated alkyl and perfluorinated $C_1$ to $C_{12}$ alkyl, perfluorinated alkoxy, perfluorinated $C_1$ to $C_6$ alkoxy or D, more preferably nitro group, CN, halogen, Cl, F, and partially fluorinated or perfluorinated $C_1$ to $C_{12}$ or D, and most preferably $NO_2$, CN, F, and perfluorinated $C_1$ to $C_{12}$ alkyl or D,

According to one embodiment of the present invention, at least one $NO_2$ is on an aryl or a heteroaryl.

According to one embodiment of the present invention, the at least one $NO_2$ is on an aryl or a heteroaryl.

According to one embodiment of the present invention, the substituent on R', R" and R'" are independently selected from electron-withdrawing group, $NO_2$, CN, halogen, Cl, F, partially fluorinated or perfluorinated alkyl and partially fluorinated or perfluorinated $C_1$ to $C_{12}$ alkyl, partially fluorinated or perfluorinated alkoxy, partially fluorinated or perfluorinated $C_1$ to $C_6$ alkoxy or D; preferably $NO_2$, CN, F, perfluorinated alkyl and perfluorinated $C_1$ to $C_{12}$ alkyl, perfluorinated alkoxy, perfluorinated $C_1$ to $C_6$ alkoxy or D. more preferably from nitro group, CN, halogen, Cl, F, and partially fluorinated or perfluorinated $C_1$ to $C_{12}$ or D, most preferably $NO_2$, CN, F, and perfluorinated $C_1$ to $C_{12}$ alkyl or D.

According to one embodiment of the present invention, R', R" and R'" are independently selected from partially fluorinated or perfluorinated $C_1$ to $C_6$ alkyl, halogen, F, CN, and $NO_2$ preferably CN and $NO_2$.

According to one embodiment of the present invention, R', R" and R'" are CN.

According to one embodiment of the present invention, $A^1$, $A^2$, and $A^3$ are represented by formula (IIa), (IIIa) and (IVa), respectively

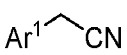 (IIa), 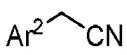 (IIIa), and 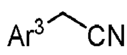 (IVa).

According to one embodiment of the present invention, $Ar^1$, $Ar^2$ and $Ar^3$ are independently selected from substituted or unsubstituted $C_6$ to $C_{18}$ aryl, substituted or unsubstituted $C_2$ to $C_{18}$ heteroaryl.

According to one embodiment of the present invention, $Ar^1$ is represented by formula (V)

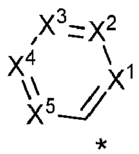 (V)

wherein $X^1$ is selected from $CR^1$ or N;

$X^2$ is selected from $CR^2$ or N;

$X^3$ is selected from $CR^3$ or N;
$X^4$ is selected from $CR^4$ or N;
$X^5$ is selected from $CR^5$ or N;
wherein one of $R^1$, $R^2$, $R^3$, $R^4$, and $R^5$ is $NO_2$ and the other $R^1$, $R^2$, $R^3$, $R^4$, and $R^5$ are independently selected from electron-withdrawing group, $NO_2$, CN, halogen, Cl, F, partially fluorinated or perfluorinated alkyl, partially fluorinated or perfluorinated $C_1$ to $C_{10}$ alkyl, partially fluorinated or perfluorinated alkoxy, partially fluorinated or perfluorinated $C_1$ to $C_6$ alkoxy, D or H;

wherein at least two of $X^1$ to $X^5$ are independently selected from $CR^1$ to $CR^5$; and wherein the asterix "*" denotes the binding position.

According to one embodiment of the present invention, $Ar^1$ is represented by formula (V)

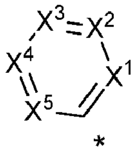 (V)

wherein $X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;
wherein one of $R^1$, $R^2$, $R^3$, $R^4$, and $R^5$ is $NO_2$ and the other $R^1$, $R^2$, $R^3$, $R^4$, and $R^5$ are independently selected from $NO_2$, CN, F, perfluorinated alkyl, perfluorinated $C_1$ to $C_{10}$ alkyl, perfluorinated alkoxy, perfluorinated $C_1$ to $C_6$ alkoxy, D or H;

wherein at least two of $X^1$ to $X^5$ are independently selected from $CR^1$ to $CR^5$; and wherein the asterix "*" denotes the binding position.

According to one embodiment of the present invention, $R^3$ is $NO_2$.

According to one embodiment of the present invention, at least one of $X^1$ to $X^5$ is N.

According to one embodiment of the present invention, at least one of $X^1$ to $X^5$ is N with the $R^x$ that is $NO_2$ in meta-position to the N.

Thus, an embodiment according to the present invention is directed to an organic compound of formula (I):

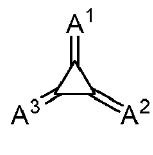

(I)

wherein $A^1$, $A^2$ and $A^3$ are represented by formula (II), (III), (IV), respectively

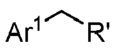 (II), 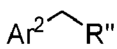 (III), and 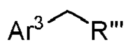 (IV)

whereby $Ar^1$ is selected from formula (V)

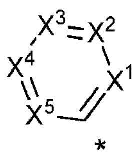 (V)

$X^1$ is selected from $CR^1$ or N;
$X^2$ is selected from $CR^2$ or N;

$X^3$ is selected from $CR^3$ or N;
$X^4$ is selected from $CR^4$ or N;
$X^5$ is selected from $CR^5$ or N;

wherein one of $R^1$, $R^2$, $R^3$, $R^4$, and $R^5$ is $NO_2$ and the other $R^1$, $R^2$, $R^3$, $R^4$, and $R^5$ are independently selected from electron-withdrawing group, $NO_2$, CN, halogen, Cl, F, partially fluorinated or perfluorinated alkyl, partially fluorinated or perfluorinated $C_1$ to $C_{10}$ alkyl, partially fluorinated or perfluorinated alkoxy, partially fluorinated or perfluorinated $C_1$ to $C_6$ alkoxy, D or H;
wherein at least two of $X^1$ to $X^5$ are independently selected from $CR^1$ to $CR^5$; and
wherein the asterix "*" denotes the binding position;
wherein $Ar^2$ and $Ar^3$ are independently selected from substituted or unsubstituted aryl, substituted or unsubstituted $C_6$ to $C_{24}$ aryl, substituted or unsubstituted heteroaryl or $C_2$ to $C_{24}$ heteroaryl, wherein the substituents on $Ar^2$ and $Ar^3$ are independently selected from an electron-withdrawing group, $NO_2$, CN, halogen, Cl, F, partially fluorinated or perfluorinated alkyl and partially fluorinated or perfluorinated $C_1$ to $C_{12}$ alkyl, partially fluorinated or perfluorinated alkoxy, partially fluorinated or perfluorinated $C_1$ to $C_6$ alkoxy or D;
R', R" and R'" are independently selected from substituted or unsubstituted aryl, substituted or unsubstituted $C_6$ to $C_{18}$ aryl, substituted or unsubstituted heteroaryl, substituted or unsubstituted $C_2$ to $C_{18}$ heteroaryl, electron-withdrawing group, partially fluorinated or perfluorinated alkyl, partially fluorinated or perfluorinated $C_1$ to $C_6$ alkyl, halogen, F, CN, and $NO_2$;
wherein the substituent on R', R" and R'" are independently selected from electron-withdrawing group, $NO_2$, CN, halogen, Cl, F, partially fluorinated or perfluorinated alkyl and partially fluorinated or perfluorinated $C_1$ to $C_{12}$ alkyl, partially fluorinated or perfluorinated alkoxy, partially fluorinated or perfluorinated $C_1$ to $C_6$ alkoxy or D; preferably from $NO_2$, CN, halogen, Cl, F, and partially fluorinated or perfluorinated $C_1$ to $C_{12}$ or D;

or a salt of a radical anion of formula (I).

Another embodiment according to the present invention is directed to an organic compound of formula (I):

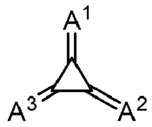

(I)

wherein $A^1$, $A^2$ and $A^3$ are represented by formula (II), (III), (IV), respectively

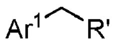 (II), 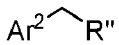 (III), and 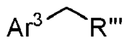 (IV)

whereby $Ar^1$ is selected from formula (V)

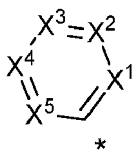

(V)

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;

wherein one of $R^1$, $R^2$, $R^3$, $R^4$, and $R^5$ is $NO_2$ and the other $R^1$, $R^2$, $R^3$, $R^4$, and $R^5$ are independently selected from $NO_2$, CN, F, perfluorinated alkyl, perfluorinated $C_1$ to $C_{10}$ alkyl, perfluorinated alkoxy, perfluorinated $C_1$ to $C_6$ alkoxy, D or H;
wherein at least two of $X^1$ to $X^5$ are independently selected from $CR^1$ to $CR^5$; and

wherein the asterix "*" denotes the binding position;

wherein $Ar^2$ and $Ar^3$ are independently selected from substituted or unsubstituted aryl, substituted or unsubstituted $C_6$ to $C_{18}$ aryl, substituted or unsubstituted heteroaryl or $C_2$ to $C_{18}$ heteroaryl, wherein the substituents on $Ar^2$ and $Ar^3$ are independently selected from $NO_2$, CN, F, partially fluorinated or perfluorinated $C_1$ to $C_{12}$ alkyl, or D;
R', R" and R'" are independently selected from substituted or unsubstituted aryl, substituted or unsubstituted $C_6$ to $C_{18}$ aryl, substituted or unsubstituted heteroaryl, substituted or unsubstituted $C_2$ to $C_{18}$ heteroaryl, partially fluorinated or perfluorinated $C_1$ to $C_6$ alkyl, F, CN, and $NO_2$;
wherein the substituent on R', R" and R'" are independently selected from $NO_2$, CN, F, perfluorinated $C_1$ to $C_{12}$ alkyl, or D;

or a salt of a radical anion of formula (I).

Another embodiment according to the present invention is directed to an organic compound of formula (I):

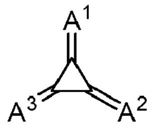

(I)

wherein $A^1$, $A^2$ and $A^3$ are represented by formula (II), (III), (IV), respectively

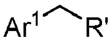 (II), 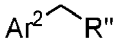 (III), and 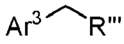 (IV)

whereby $Ar^1$ is selected from formula (V)

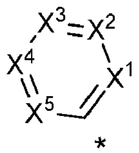

(V)

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;

wherein one of $R^1$, $R^2$, $R^3$, $R^4$, and $R^5$ is $NO_2$ and the other $R^1$, $R^2$, $R^3$, $R^4$, and $R^5$ are independently selected from $NO_2$, CN, F, perfluorinated alkyl, perfluorinated $C_1$ to $C_{10}$ alkyl, perfluorinated alkoxy, perfluorinated $C_1$ to $C_6$ alkoxy, D or H;
wherein at least two of $X^1$ to $X^5$ are independently selected from $CR^1$ to $CR^5$; and wherein the asterix "*" denotes the binding position;

wherein $Ar^2$ and $Ar^3$ are independently selected from substituted or unsubstituted aryl, substituted or unsubstituted $C_6$ to $C_{18}$ aryl, substituted or unsubstituted heteroaryl or $C_2$ to $C_{18}$ heteroaryl, wherein the substituents on $Ar^2$ and $Ar^3$ are independently selected from an electron-withdrawing group, $NO_2$, CN, F, partially fluorinated or perfluorinated $C_1$ to $C_{12}$ alkyl, or D;
R', R" and R''' are independently selected CN or $NO_2$;

or a salt of a radical anion of formula (I).

Another embodiment according to the present invention is directed to an organic compound of formula (I):

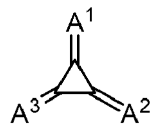

(I)

wherein $A^1$, $A^2$ and $A^3$ are represented by formula (II), (III), (IV), respectively

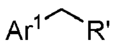 (II), 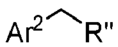 (III), and 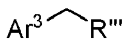 (IV)

whereby $Ar^1$ is selected from formula (V)

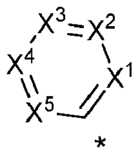 (V)

$X^1$ is selected from $CR^1$ or N;
$X^2$ is selected from $CR^2$ or N;
$X^3$ is selected from $CR^3$ or N;
$X^4$ is selected from $CR^4$ or N;
$X^5$ is selected from $CR^5$ or N;

wherein one of $R^1$, $R^2$, $R^3$, $R^4$, and $R^5$ is $NO_2$ and the other $R^1$, $R^2$, $R^3$, $R^4$, and $R^5$ are independently selected from $NO_2$, CN, F, perfluorinated alkyl, perfluorinated $C_1$ to $C_{10}$ alkyl, perfluorinated alkoxy, perfluorinated $C_1$ to $C_6$ alkoxy, D or H;
wherein at least two of $X^1$ to $X^5$ are independently selected from $CR^1$ to $CR^5$; and wherein the asterix "*" denotes the binding position;

wherein $Ar^2$ and $Ar^3$ are independently selected from substituted or unsubstituted aryl, substituted or unsubstituted $C_6$ to $C_{18}$ aryl, substituted or unsubstituted heteroaryl or $C_2$ to $C_{18}$ heteroaryl, wherein the substituents on $Ar^2$ and $Ar^3$ are independently selected from an electron-withdrawing group, $NO_2$, CN, F, partially fluorinated or perfluorinated $C_1$ to $C_{12}$ alkyl, or D;
R', R" and R''' are CN;

or a salt of a radical anion of formula (I).

According to one embodiment of the present invention, the compound is selected of the formula (VI)

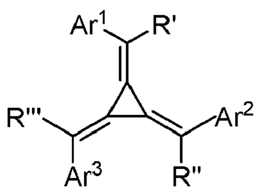

(VI)

According to one embodiment of the present invention, at least one of $Ar^2$ and $Ar^3$ is substituted with at least one $NO_2$.

According to one embodiment of the present invention, at least one of $Ar^2$ and $Ar^3$ does not contain hydrogen.

According to one embodiment of the present invention, at least two of $Ar^1$, $Ar^2$ and $Ar^3$ are identical.

According to one embodiment of the present invention, $Ar^2$ and $Ar^3$ are identical.

According to one embodiment of the present invention, $Ar^1$, $Ar^2$ and $Ar^3$ are identical.

According to one embodiment of the present invention, whereby $Ar^2$ is represented by formula (VII) and $Ar^3$ is represented by formula (VIII):

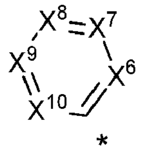

(VII)

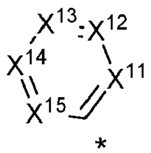

(VIII)

wherein $X^6$ is selected from $CR^6$ or N;
$X^7$ is selected from $CR^7$ or N;
$X^8$ is selected from $CR^8$ or N;
$X^9$ is selected from $CR^9$ or N;
$X^{10}$ is selected from $CR^{10}$ or N;
wherein $X^{11}$ is selected from $CR^{11}$ or N;
$X^{12}$ is selected from $CR^{12}$ or N;
$X^{13}$ is selected from $CR^{13}$ or N;
$X^{14}$ is selected from $CR^{14}$ or N;
$X^{15}$ is selected from $CR^{15}$ or N;
wherein $R^6$, $R^7$, $R^8$, $R^9$, $R^{10}$, $R^{11}$, $R^{12}$, $R^{13}$, $R^{14}$, and $R^{15}$ are independently selected from electron-withdrawing group, $NO_2$, CN, halogen, Cl, F, partially fluorinated or perfluorinated alkyl, partially fluorinated or perfluorinated $C_1$ to $C_{10}$ alkyl, partially fluorinated or perfluorinated alkoxy, partially fluorinated or perfluorinated $C_1$ to $C_6$ alkoxy, D or H, preferably $NO_2$, CN, halogen, Cl, F, partially fluorinated or perfluorinated $C_1$ to $C_6$ alkyl, D or H;

wherein at least two of $X^6$ to $X^{10}$ are independently selected from $CR^6$ to $CR^{10}$; wherein at least two of $X^{11}$ to $X^{15}$ are independently selected from $CR^{11}$ to $CR^{15}$; and wherein the asterix "*" denotes the binding position.

According to one embodiment of the present invention, whereby $Ar^2$ is represented by formula (VII) and $Ar^3$ is represented by formula (VIII):

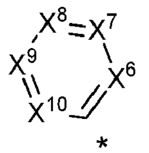

(VII)

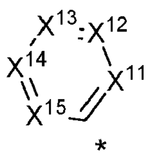

(VIII)

wherein $X^6$ is selected from $CR^6$ or N;
$X^7$ is selected from $CR^7$ or N;
$X^8$ is selected from $CR^8$ or N;
$X^9$ is selected from $CR^9$ or N;
$X^{10}$ is selected from $CR^{10}$ or N;
wherein $X^{11}$ is selected from $CR^{11}$ or N;
$X^{12}$ is selected from $CR^{12}$ or N;
$X^{13}$ is selected from $CR^{13}$ or N;
$X^{14}$ is selected from $CR^{14}$ or N;
$X^{15}$ is selected from $CR^{15}$ or N;
wherein one $R^6$, $R^7$, $R^8$, $R^9$, and $R^{10}$ is $NO_2$ and the other $R^6$, $R^7$, $R^8$, $R^9$, $R^{10}$ are independently selected from

electron-withdrawing group, $NO_2$, CN, halogen, Cl, F, partially fluorinated or perfluorinated alkyl, partially fluorinated or perfluorinated $C_1$ to $C_{10}$ alkyl, partially fluorinated or perfluorinated alkoxy, partially fluorinated or perfluorinated $C_1$ to $C_6$ alkoxy, D or H, preferably $NO_2$, CN, halogen, Cl, F, partially fluorinated or perfluorinated $C_1$ to $C_6$ alkyl, D or H, more preferably $NO_2$, CN, F, perfluorinated alkyl, perfluorinated $C_1$ to $C_{10}$ alkyl, perfluorinated alkoxy, perfluorinated $C_1$ to $C_6$ alkoxy, D or H;

wherein one of $R^{11}$, $R^{12}$, $R^{13}$, $R^{14}$, and $R^{15}$ is $NO_2$ and the other $R^{11}$, $R^{12}$, $R^{13}$, $R^{14}$, and $R^{15}$ are independently selected from electron-withdrawing group, $NO_2$, CN, halogen, Cl, F, partially fluorinated or perfluorinated alkyl, partially fluorinated or perfluorinated $C_1$ to $C_{10}$ alkyl, partially fluorinated or perfluorinated alkoxy, partially fluorinated or perfluorinated $C_1$ to $C_6$ alkoxy, D or H, preferably $NO_2$, CN, halogen, Cl, F, partially fluorinated or perfluorinated $C_1$ to $C_6$ alkyl, D or H, more preferably $NO_2$, CN, F, perfluorinated alkyl, perfluorinated $C_1$ to $C_{10}$ alkyl, perfluorinated alkoxy, perfluorinated $C_1$ to $C_6$ alkoxy, D or H;

wherein at least two of $X^6$ to $X^{10}$ are independently selected from $CR^6$ to $CR^{10}$; wherein at least two of $X^{11}$ to $X^{15}$ are independently selected from $CR^{11}$ to $CR^{15}$; and wherein the asterix "*" denotes the binding position.

According to one embodiment of the present invention, the compound contains four or less hydrogen atoms.

According to one embodiment of the present invention, the compound contains at least eight fluorine atoms, preferably at least ten fluorine atoms.

According to one embodiment of the present invention, $Ar^2$ and $Ar^3$ each comprise only one moiety selected out of $CF_3$, CN.

According to one embodiment of the present invention, at least one of $Ar^1$, $Ar^2$ and $Ar^3$, preferably $Ar^1$ or at least $Ar^1$ is selected from one of the following moieties:

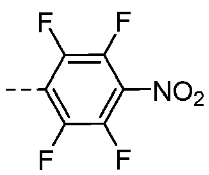 , 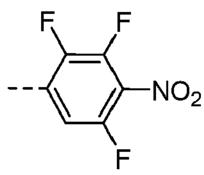 , 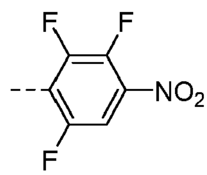 ,

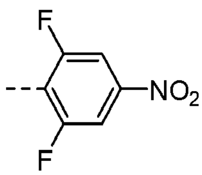 , 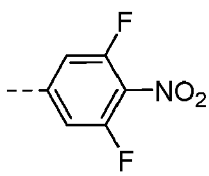 , 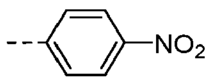 ,

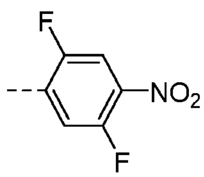 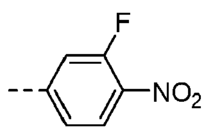 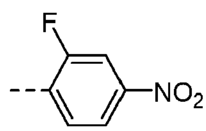

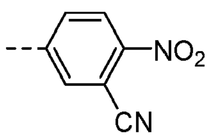 , 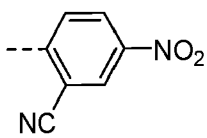 , 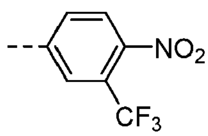 ,

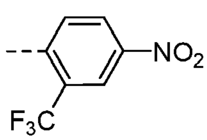 , 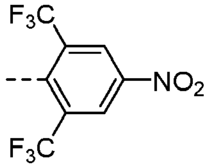 , 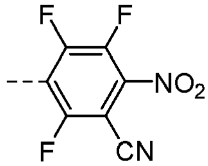 ,

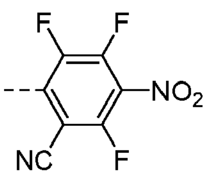 , 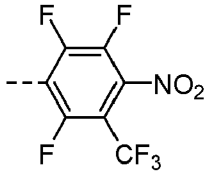 , 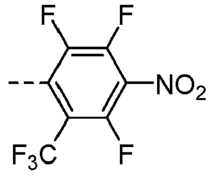 ,

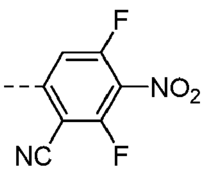 , 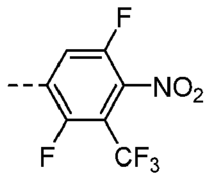 , 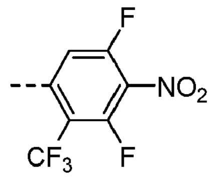 ,

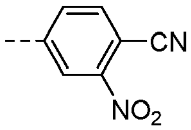 , 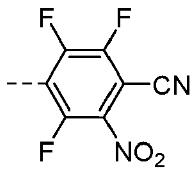 , 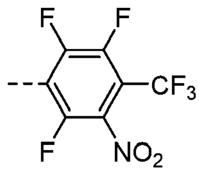 ,

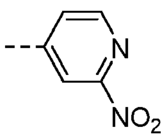 , 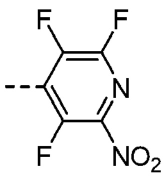 , 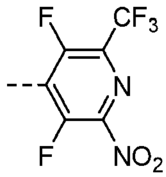 ,

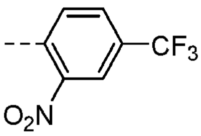 , 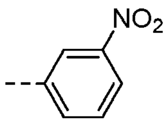 , 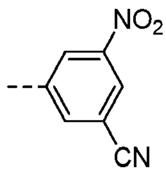 ,

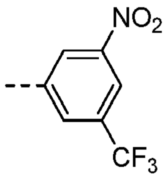 , 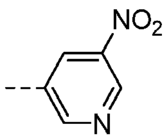 , 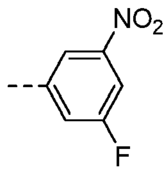 ,

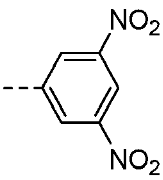 , 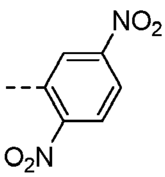 , 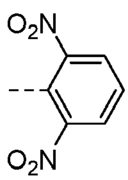 ,

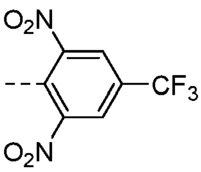 , 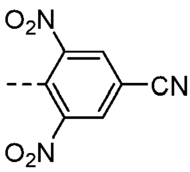 , 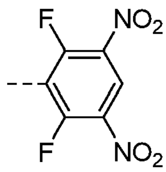 ,

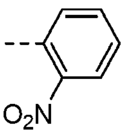 , 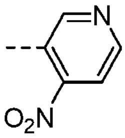 , 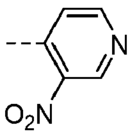 ,

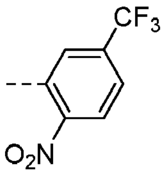 , 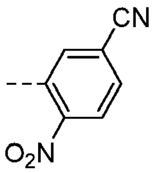 , 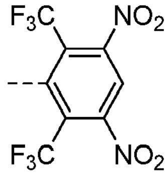 ,

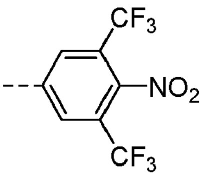 , 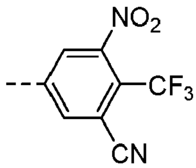 , 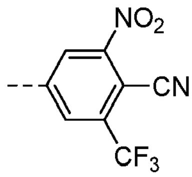 ,

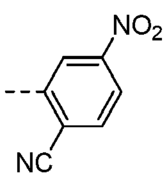 , 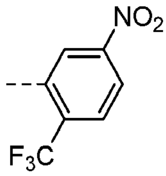 , 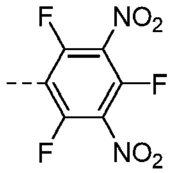 ,

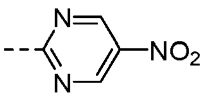 , 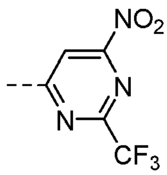 , 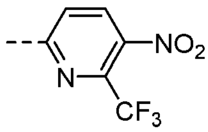 ,

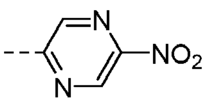 , 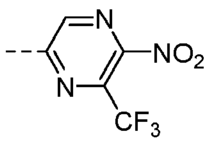 , 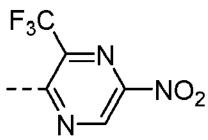 ,

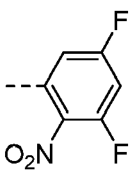 , 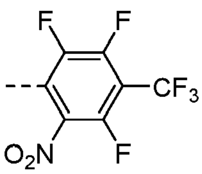 , 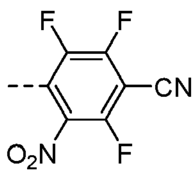 ,

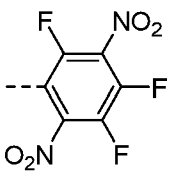 , 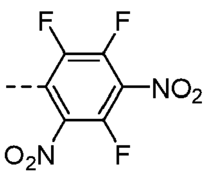 , 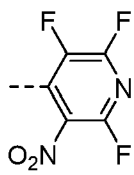 ,

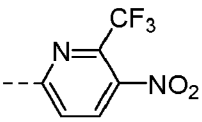,
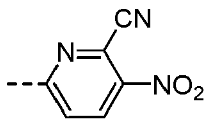,
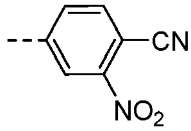,
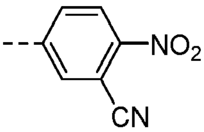,
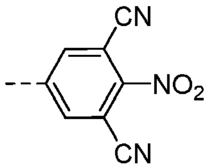,
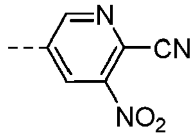,
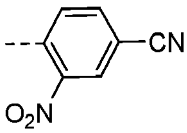,
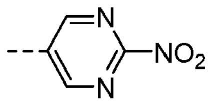, and
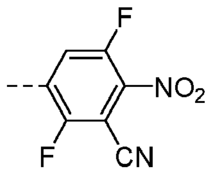.

According to one embodiment of the present invention, at least two of $A^1$, $A^2$, and $A^3$ are identical.

According to one embodiment of the present invention, $A^2$ and $A^3$ are identical.

According to one embodiment of the present invention, $A^1$, $A^2$, and $A^3$ are identical.

According to one embodiment of the present invention, $A^2$ and $A^3$ are independently selected from

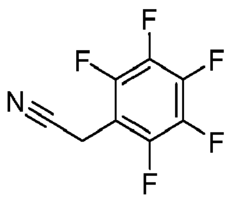,
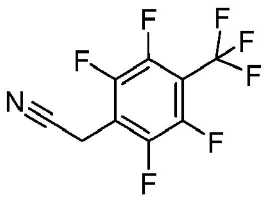,
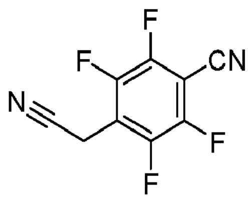,
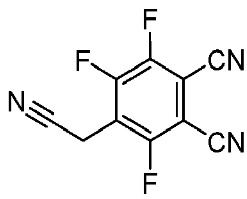,
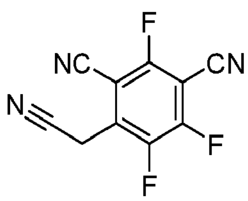
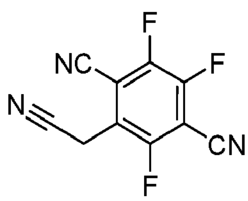,
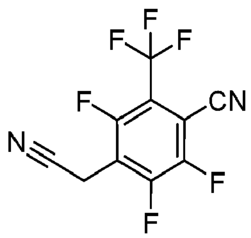,
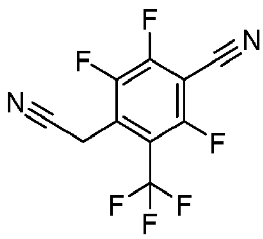,
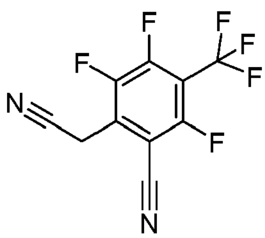,
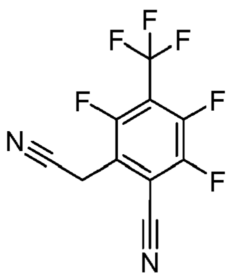,
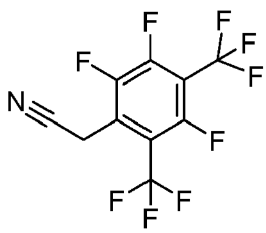,
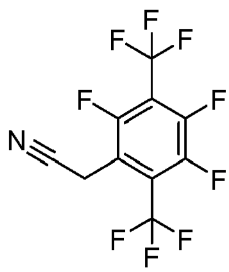,

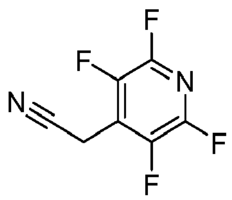
, s
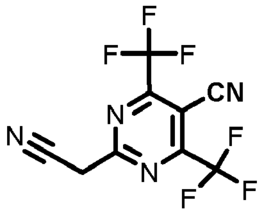
,
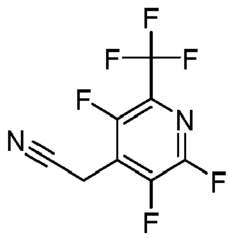
,
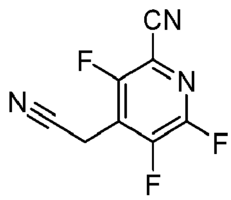
,

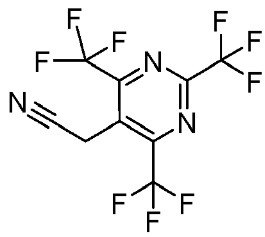
,
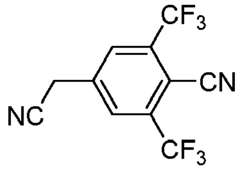
,
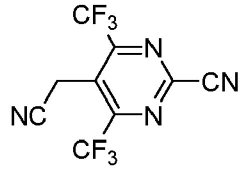
,
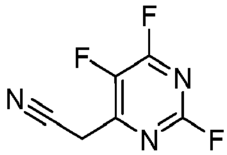
,

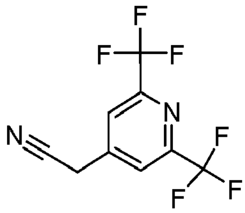
,
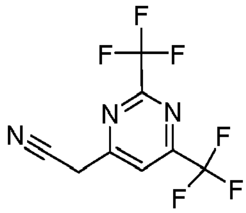
,
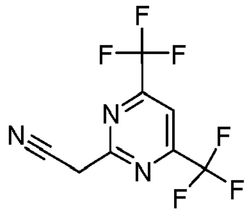
,
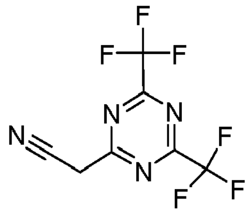
,

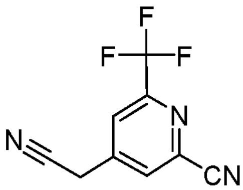
,
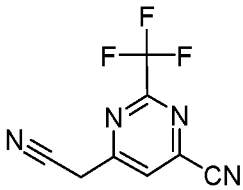
,
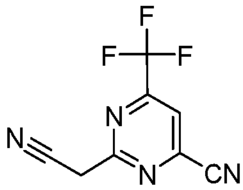
,
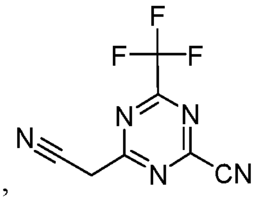
,

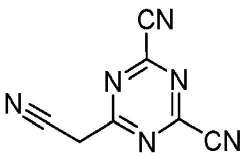
,
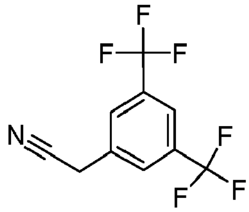
,
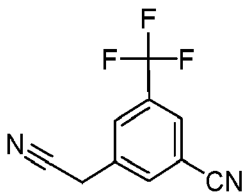
,
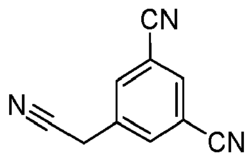
,

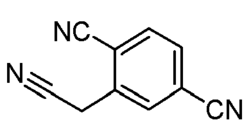
,
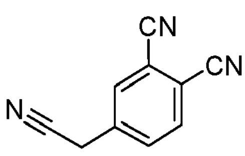
,
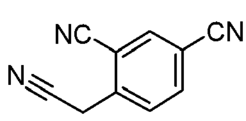
,
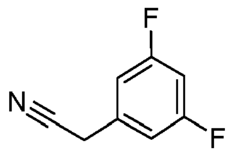
,

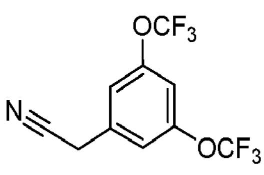
,
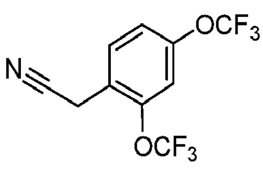
,
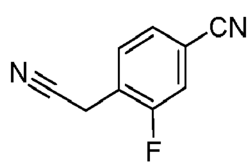
,
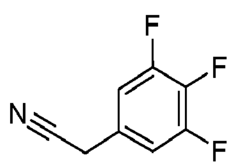
,

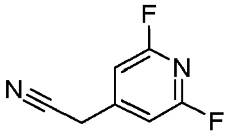 . and 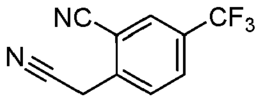 .

According to one embodiment of the present invention, at least one of $A^1$, $A^2$ and $A^3$, preferably $A^1$ or at least $A^1$ is selected from one of the following moieties:

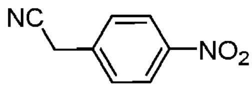 , 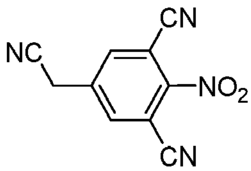 , 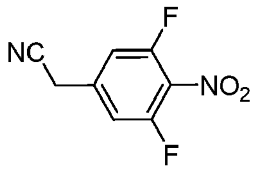 ,

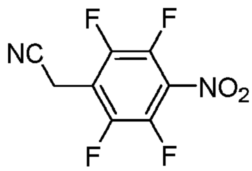 , 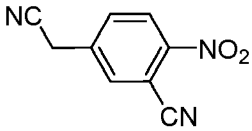 , 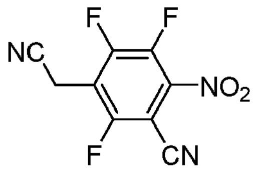 ,

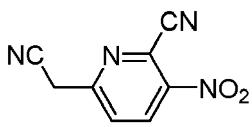 , 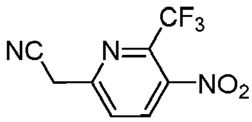 , 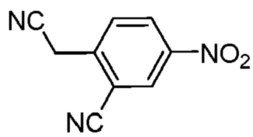 ,

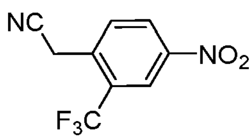 , 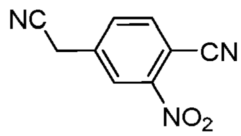 , 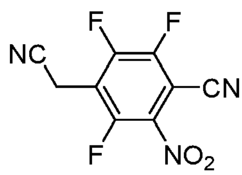 ,

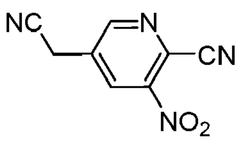 , and 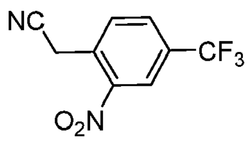 .

According to one embodiment of the present invention, the compound is selected from one of the following compounds C1-C25, whereby R', R" and R''' are CN and $Ar^1$, $Ar^2$ and $Ar^3$ are selected from the following table:

| Compound | $Ar^1$ | $Ar^2$ | $Ar^3$ |
|---|---|---|---|
| C1 | 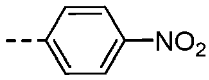 | 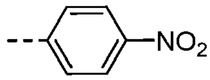 | 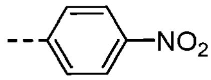 |
| C2 | 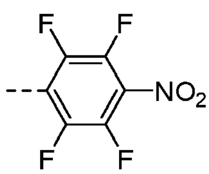 | 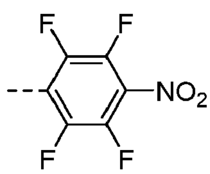 | 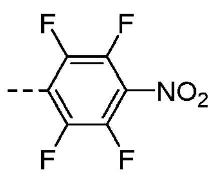 |

(continued)

| Compound | $Ar^1$ | $Ar^2$ | $Ar^3$ |
|---|---|---|---|
| C3 | 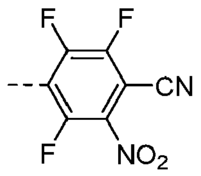 | 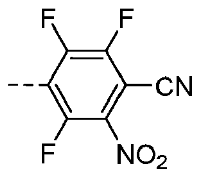 | 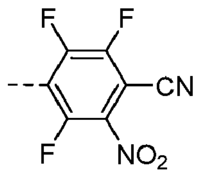 |
| C4 | 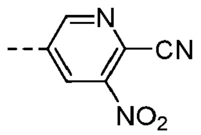 | 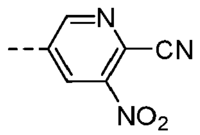 | 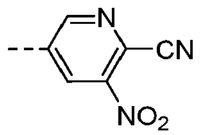 |
| C5 | 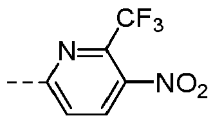 | 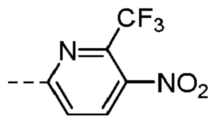 | 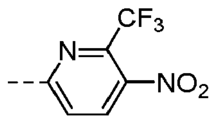 |
| C6 | 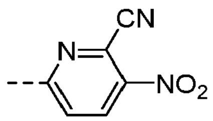 | 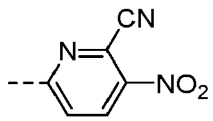 | 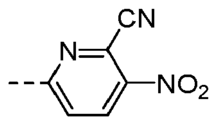 |
| C7 | 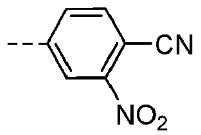 | 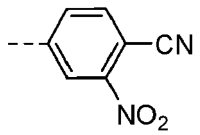 | 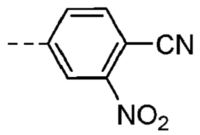 |
| C8 | 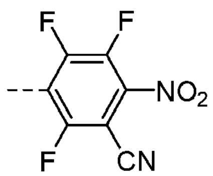 | 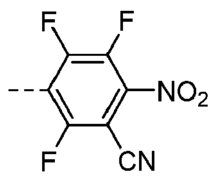 | 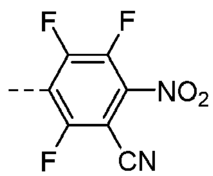 |
| C9 | 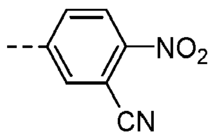 | 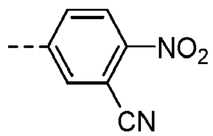 | 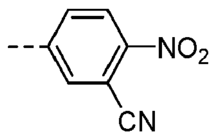 |
| C10 | 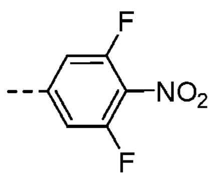 | 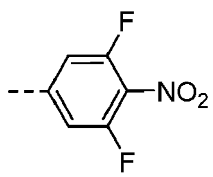 | 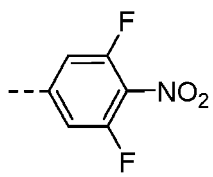 |
| C11 | 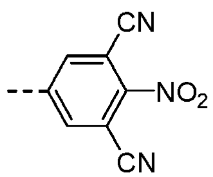 | 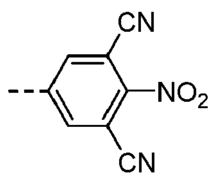 | 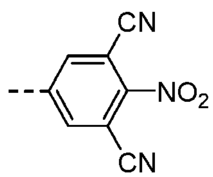 |
| C12 | 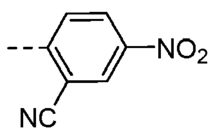 | 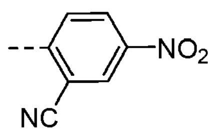 | 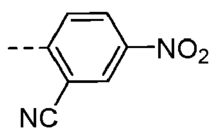 |

(continued)

| Compound | Ar1 | Ar2 | Ar3 |
|---|---|---|---|
| C13 | 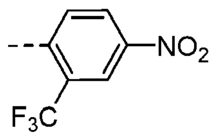 | 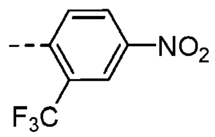 | 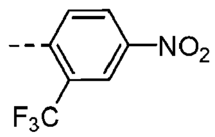 |
| C14 | 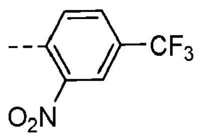 | 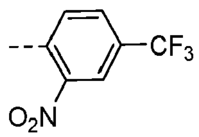 | 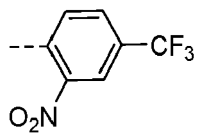 |
| C15 | 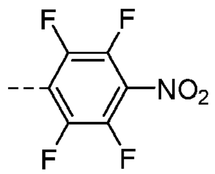 | 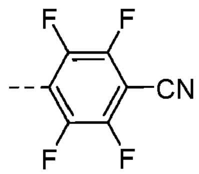 | 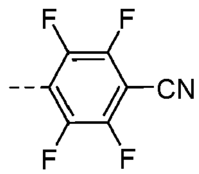 |
| C16 | 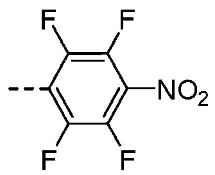 | 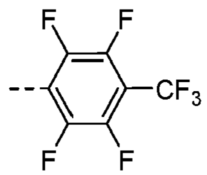 | 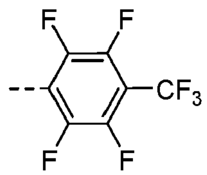 |
| C17 | 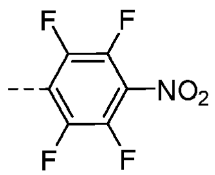 | 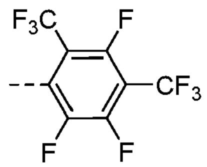 | 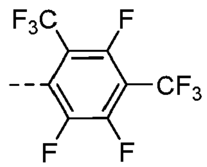 |
| C18 | 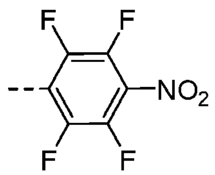 | 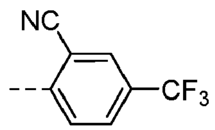 | 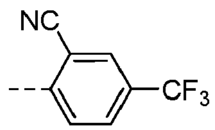 |
| C19 | 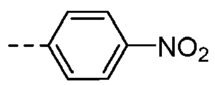 | 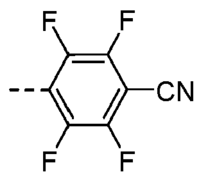 | 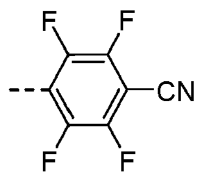 |
| C20 | 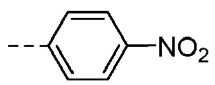 | 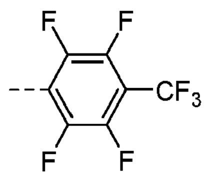 | 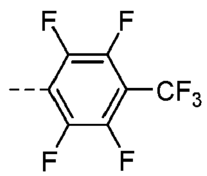 |
| C21 | 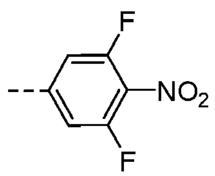 | 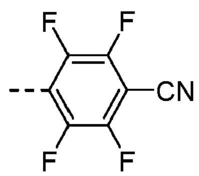 | 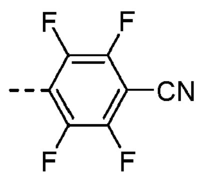 |

(continued)

| Compound | $Ar^1$ | $Ar^2$ | $Ar^3$ |
|---|---|---|---|
| C22 | 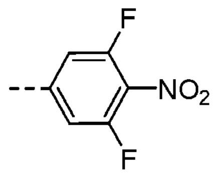 | 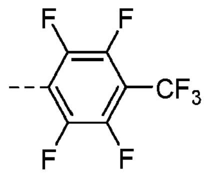 | 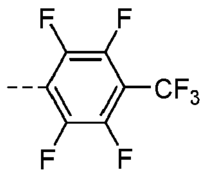 |
| C23 | 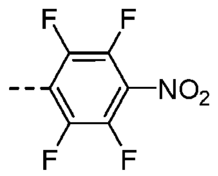 | 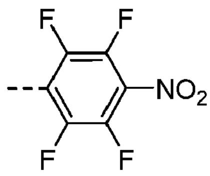 | 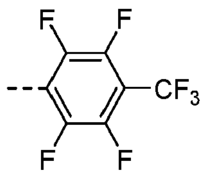 |
| C24 | 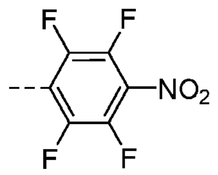 | 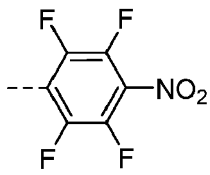 | 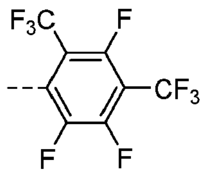 |
| C25 | 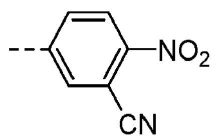 | 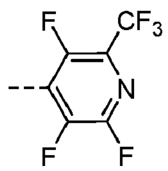 | 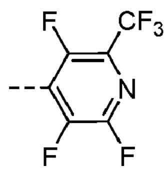 |

According to one embodiment of the present invention, the LUMO level of compound of formula (I) is selected in the range of ≤ -4.4 eV and ≥ -5.9 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase, preferably in the range of ≤ -4.5 eV and ≥ -5.5 eV and most preferred in the range of ≤ -4.7 eV and ≥ -5.25 eV.

According to one embodiment of the present invention, wherein in compound of formula (I) $A^1$, $A^2$ and $A^3$ may be selected the same and the LUMO level of compound of formula (I) is selected in the range of ≤ -4.7 eV and ≥ -5.9 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase, preferably in the range of ≤ -4.72 eV and ≥ -5.5 eV and most preferred in the range of ≤ -4.75 eV and ≥ -5.25 eV.

According to one embodiment of the present invention, wherein in compound of formula (I) $A^1$ and $A^2$ may be selected the same or differently and $A^1$ is not identical to $A^3$ and the LUMO level of compound of formula (I) is selected in the range of ≤ -4.5 eV and ≥ -5.4 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase, preferably in the range of ≤ -4.6 eV and ≥ -5.25 eV and most preferred in the range of ≤ -4.7 eV and ≥ -5.25 eV.

According to one embodiment of the present invention, wherein the ratio between the amount of atoms (without $NO_2$) to the amount of $NO_2$ is at least 6:1, preferably 8:1, and more preferably 10:1.

According to one embodiment of the present invention, wherein the ratio between the amount of carbon atoms to the amount of $NO_2$ is at least 6:1, preferably 8:1, and more preferably 10:1.

The present invention furthermore relates to a composition comprising a compound of formula (VI) and at least one compound of formula (VIa) to (VId)

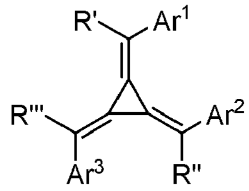

(VIa)

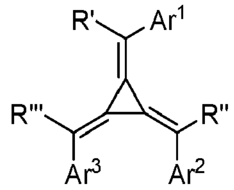

(VIb)

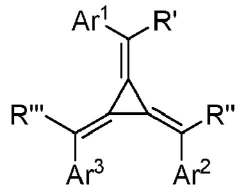

(VIc)

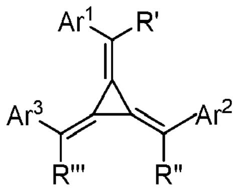

(VId)

Another aspect of the present invention is related to an organic semiconductor layer comprising at least one compound of formula (I).

The present invention is also related to an organic electronic device comprising at least one compound of formula (I)

Another aspect of the present invention is related to an organic semiconductor layer comprising an organic semiconductor layer.

The present invention furthermore relates to an organic electronic device comprising an anode layer, a cathode layer and at least one organic semiconductor layer, wherein the at least one organic semiconductor layer comprises a compound of formula (I).

According to one embodiment, the organic electronic device comprising an anode layer, a cathode layer.

According to one embodiment, the organic electronic device further comprises a photoactive layer.

According to one embodiment, the organic electronic device comprising an anode layer, a cathode layer and at least one organic semiconductor layer, wherein the at least one organic semiconductor layer is arranged between the anode layer and the cathode layer; and wherein the at least one organic semiconductor layer comprises a compound of formula (I).

According to one embodiment, the organic semiconductor layer comprises a composition comprising a compound of formula (VI) and at least one compound of formula (VIa) to (VId) as described above.

In case the organic semiconductor layer comprises such a composition, throughout this application text the term "compound of formula (I)" shall also intend to include the composition as described above.

Another aspect of the present invention provides an organic electronic device comprising an anode layer, a cathode layer, at least one organic semiconductor layer, and at least one photoactive layer, wherein the semiconductor layer is arranged between the cathode and the at least one photoactive layer, wherein the at least one organic semiconductor layer comprises a compound of formula (I).

Another aspect of the present invention provides an organic electronic device comprising an anode layer, a cathode layer and a charge generation layer, wherein the charge generation layer comprises a p-type charge generation layer and a n-type charge generation layer, wherein the p-type charge generation layer comprises a compound of formula

(I).

According to one embodiment of the present invention the organic semiconductor layer and/or the compound of formula (I) are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

According to one embodiment of the invention, the at least one organic semiconductor layer further comprises a substantially covalent matrix compound. Preferably, at least one semiconductor layer further comprising a substantially covalent matrix compound is arranged and/or provided adjacent to the anode layer.

Substantially covalent matrix compound

The organic semiconductor layer may further comprises a substantially covalent matrix compound. According to one embodiment the substantially covalent matrix compound may be selected from at least one organic compound. The substantially covalent matrix may consists substantially from covalently bound C, H, O, N, S, which optionally comprise in addition covalently bound B, P, As and/or Se.

According to one embodiment of the organic electronic device, the organic semiconductor layer further comprises a substantially covalent matrix compound, wherein the substantially covalent matrix compound may be selected from organic compounds consisting substantially from covalently bound C, H, O, N, S, which optionally comprise in addition covalently bound B, P, As and/or Se.

Organometallic compounds comprising covalent bonds carbon-metal, metal complexes comprising organic ligands and metal salts of organic acids are further examples of organic compounds that may serve as substantially covalent matrix compounds of the hole injection layer.

In one embodiment, the substantially covalent matrix compound lacks metal atoms and majority of its skeletal atoms may be selected from C, O, S, N. Alternatively, the substantially covalent matrix compound lacks metal atoms and majority of its skeletal atoms may be selected from C and N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound comprises at least one arylamine moiety, alternatively a diarylamine moiety, alternatively a triarylamine moiety.

Preferably, the substantially covalent matrix compound is free of metals and/or ionic bonds.

Compound of formula (IX) or a compound of formula (X)

According to another aspect of the present invention, the at least one matrix compound, also referred to as "substantially covalent matrix compound", may comprises at least one arylamine compound, diarylamine compound, triarylamine compound, a compound of formula (IX) or a compound of formula (X):

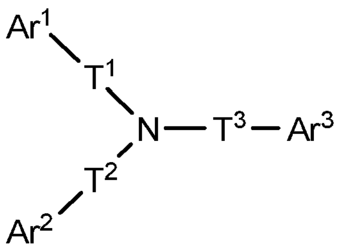 (IX), 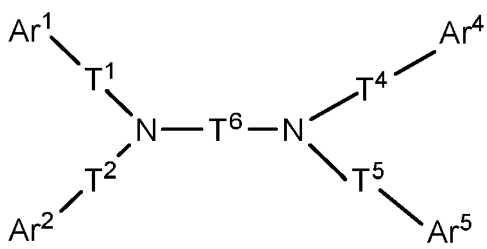 (X),

wherein:

$T^1$, $T^2$, $T^3$, $T^4$ and $T^5$ are independently selected from a single bond, phenylene, biphenylene, terphenylene or naphthenylene, preferably a single bond or phenylene;

$T^6$ is phenylene, biphenylene, terphenylene or naphthenylene;

$Ar^1$, $Ar^2$, $Ar^3$, $Ar^4$ and $Ar^5$ are independently selected from substituted or unsubstituted $C_6$ to $C_{20}$ aryl, or substituted or unsubstituted $C_3$ to $C_{20}$ heteroarylene, substituted or unsubstituted biphenylene, substituted or unsubstituted

fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;

wherein

the substituents of $Ar^1$, $Ar^2$, $Ar^3$, $Ar^4$ and $Ar^5$ are selected the same or different from the group comprising H, D, F, $C(-O)R^2$, CN, $Si(R^2)_3$, $P(-O)(R^2)_2$, $OR^2$, $S(-O)R^2$, $S(-O)_2R^2$, substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted $C_6$ to $C_{18}$ aryl, unsubstituted $C_3$ to $C_{18}$ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle, wherein $R^2$ may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, $C_6$ to $C_{18}$ aryl or $C_3$ to $C_{18}$ heteroaryl.

According to an embodiment wherein $T^1$, $T^2$, $T^3$, $T^4$ and $T^5$ may be independently selected from a single bond, phenylene, biphenylene or terphenylene. According to an embodiment wherein $T^1$, $T^2$, $T^3$, $T^4$ and $T^5$ may be independently selected from phenylene, biphenylene or terphenylene and one of $T^1$, $T^2$, $T^3$, $T^4$ and $T^5$ are a single bond. According to an embodiment wherein $T^1$, $T^2$, $T^3$, $T^4$ and $T^5$ may be independently selected from phenylene or biphenylene and one of $T^1$, $T^2$, $T^3$, $T^4$ and $T^5$ are a single bond. According to an embodiment wherein $T^1$, $T^2$, $T^3$, $T^4$ and $T^5$ may be independently selected from phenylene or biphenylene and two of $T^1$, $T^2$, $T^3$, $T^4$ and $T^5$ are a single bond.

According to an embodiment wherein $T^1$, $T^2$ and $T^3$ may be independently selected from phenylene and one of $T^1$, $T^2$ and $T^3$ are a single bond. According to an embodiment wherein $T^1$, $T^2$ and $T^3$ may be independently selected from phenylene and two of $T^1$, $T^2$ and $T^3$ are a single bond.

According to an embodiment wherein $T^6$ may be phenylene, biphenylene, terphenylene. According to an embodiment wherein $T^6$ may be phenylene. According to an embodiment wherein $T^6$ may be biphenylene. According to an embodiment wherein $T^6$ may be terphenylene.

According to an embodiment wherein $Ar^1$, $Ar^2$, $Ar^3$, $Ar^4$ and $Ar^5$ may be independently selected from D1 to D16:

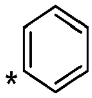 (D1), 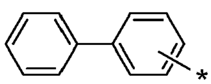 (D2), 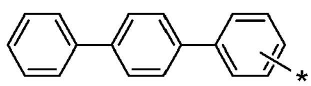 (D3), 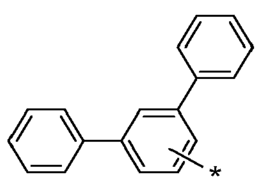 (D4),

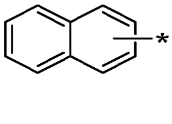 (D5), 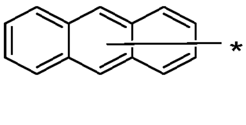 (D6), 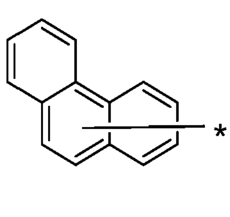 (D7), 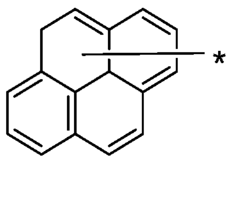 (D8),

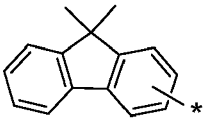 (D9), 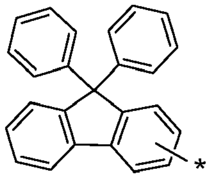 (D10), 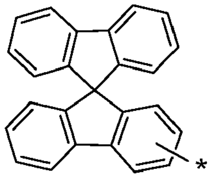 (D11),

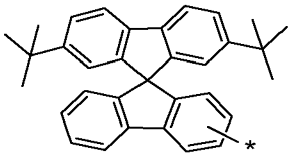 (D12), 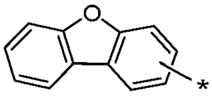 (D13), 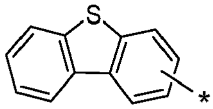 (D14),

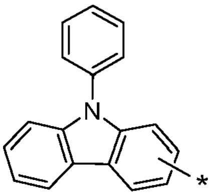 (D15), 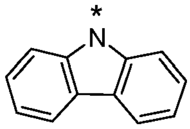 (D16),

wherein the asterix "*" denotes the binding position.

According to an embodiment, wherein $Ar^1$, $Ar^2$, $Ar^3$, $Ar^4$ and $Ar^5$ may be independently selected from D1 to D15; alternatively selected from D1 to D10 and D13 to D15.

According to an embodiment, wherein $Ar^1$, $Ar^2$, $Ar^3$, $Ar^4$ and $Ar^5$ may be independently selected from the group consisting of D1, D2, D5, D7, D9, D10, D13 to D16.

The rate onset temperature may be in a range particularly suited to mass production, when $Ar^1$, $Ar^2$, $Ar^3$, $Ar^4$ and $Ar^5$ are selected in this range.

The "matrix compound of formula (IX) or formula (X)" may be also referred to as "hole transport compound".

According to one embodiment, the substantially covalent matrix compound comprises at least one naphthyl group, carbazole group, dibenzofuran group, dibenzothiophene group and/or substituted fluorenyl group, wherein the substituents are independently selected from methyl, phenyl or fluorenyl.

According to an embodiment of the electronic device, wherein the matrix compound of formula (IX) or formula (X) are selected from F1 to F18:

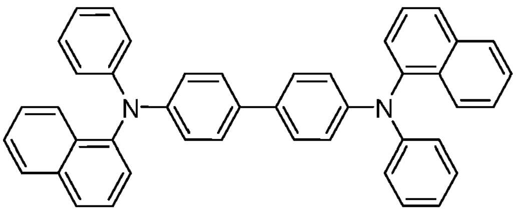 (F1), 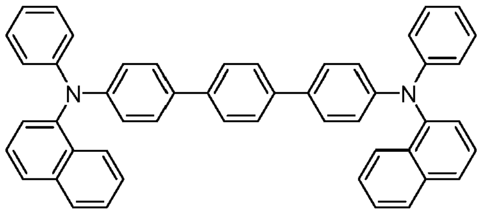 (F2),

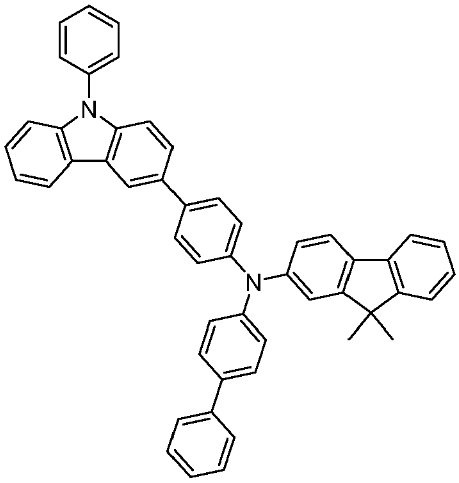 (F3), 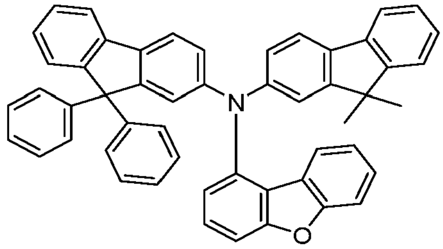 (F4),

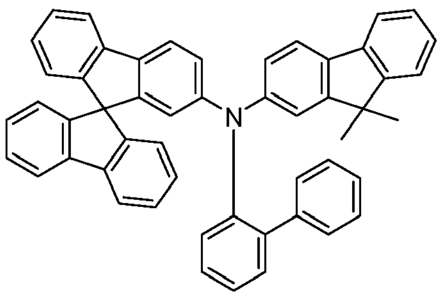 (F5), 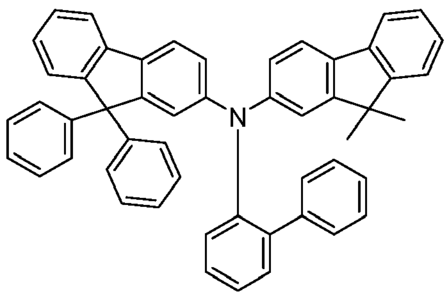 (F6),

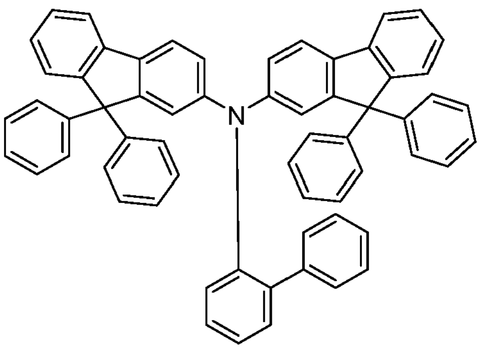 (F7), 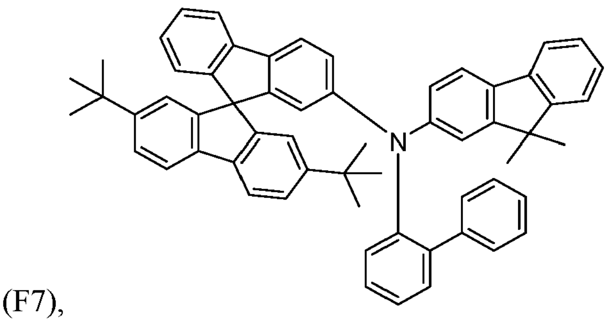 (F8),

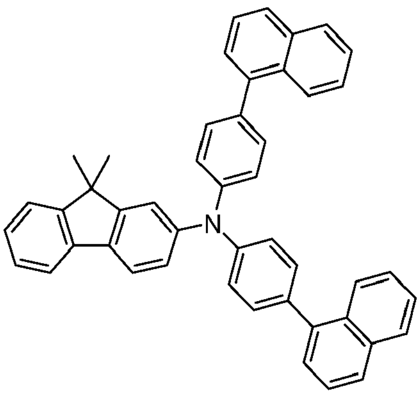 (F9), 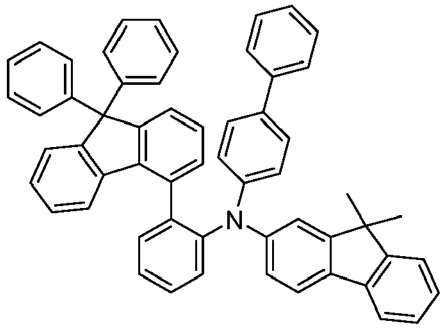 (F10),

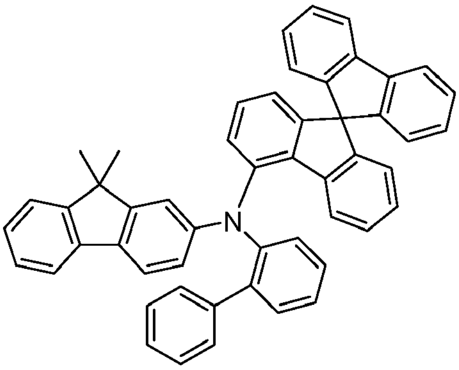 (F11), 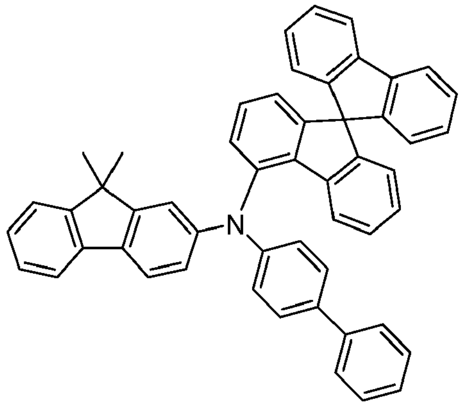 (F12),

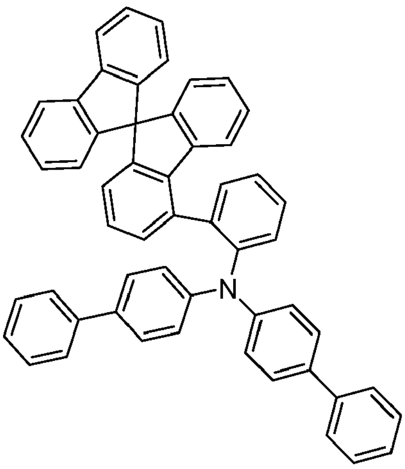 (F13), 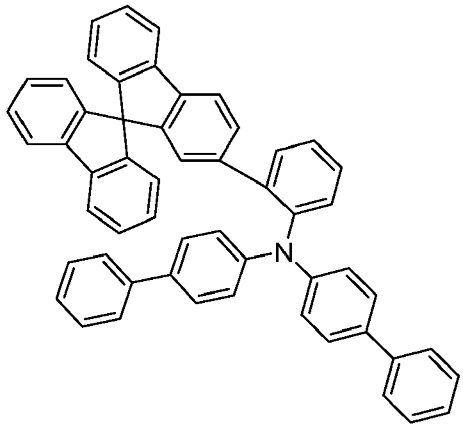 (F14),

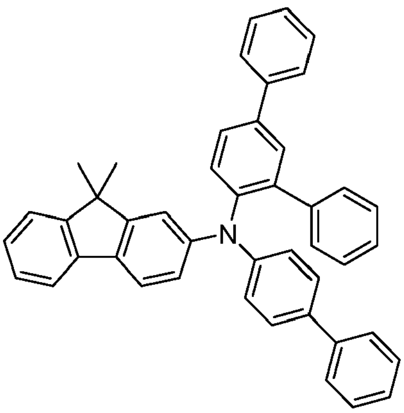 (F15), 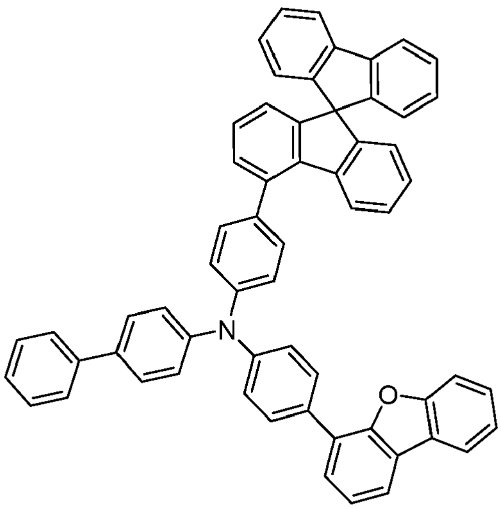 (F16),

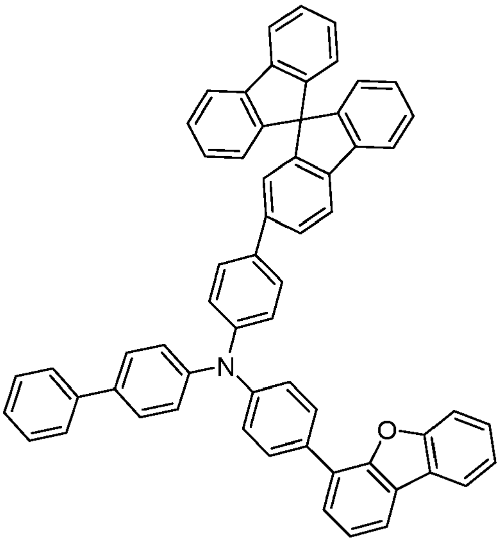

(F17)

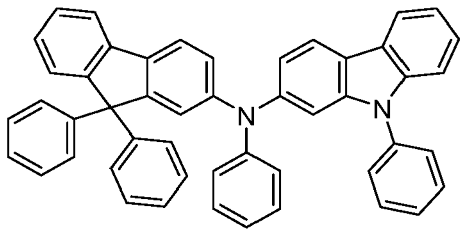

(F18).

Organic semiconductor layer

The organic semiconductor layer may be formed on the anode layer or cathode layer by vacuum deposition, spin coating, printing, casting, slot-die coating, Langmuir-Blodgett (LB) deposition, or the like. When the Organic semiconductor layer is formed using vacuum deposition, the deposition conditions may vary according to the compound(s) that are used to form the layer, and the desired structure and thermal properties of the layer. In general, however, conditions for vacuum deposition may include a deposition temperature of 100° C to 350° C, a pressure of $10^{-8}$ to $10^{-3}$ Torr (1 Torr equals 133.322 Pa), and a deposition rate of 0.1 to 10 nm/sec.

When the organic semiconductor layer is formed using spin coating or printing, coating conditions may vary according to the compound(s) that are used to form the layer, and the desired structure and thermal properties of the organic semiconductor layer. For example, the coating conditions may include a coating speed of about 2000 rpm to about 5000 rpm, and a thermal treatment temperature of about 80° C to about 200° C. Thermal treatment removes a solvent after the coating is performed.

The thickness of the organic semiconductor layer may be in the range from about 1 nm to about 20 nm, and for example, from about 2 nm to about 15 nm, alternatively about 2 nm to about 12 nm.

When the thickness of the organic semiconductor layer is within this range, the organic semiconductor layer may have excellent hole injecting and/or hole generation characteristics, without a substantial penalty in driving voltage.

According to one embodiment of the present invention, the organic semiconductor layer may comprise:

- at least about ≥ 0.5 wt.-% to about ≤ 30 wt.-%, preferably about ≥ 0.5 wt.-% to about ≤ 20 wt.-%, and more preferred about ≥ 1 wt.-% to about ≤ 15 wt.-% of a compound of formula (I), and
- at least about ≥ 70 wt.-% to about ≤ 99.5 wt.-%, preferably about ≥ 80 wt.-% to about ≤ 99.5 wt.-%, and more preferred about ≥ 85 wt.-% to about ≤ 99 wt.-% of a substantially covalent matrix compound; preferably the wt.-% of the compound of formula (I) is lower than the wt.-% of the substantially covalent matrix compound; wherein the weight-% of the components are based on the total weight of the organic semiconductor layer.

According to one embodiment of the invention, the organic electronic devices comprises at least one photoactive layer, wherein the photoactive layer is arranged between the anode layer and the cathode layer.

According to one embodiment of the invention, the organic electronic devices comprises at least one photoactive layer and at least one organic semiconductor layer is arranged between the anode and at least one photoactive layer.

According to one embodiment of the invention, the organic electronic devices comprises at least one photoactive layer and the at least one organic semiconductor layer is arranged between the anode and at least one photoactive layer.

According to one embodiment of the invention, the organic electronic devices comprises at least one photoactive layer and the at least one organic semiconductor layer is arranged between the anode and the at least one photoactive layer.

According to one embodiment of the invention, the organic electronic devices comprises at least one photoactive layer and at least one of the at least one organic semiconductor layer is arranged between the anode and at least one of the at least one photoactive layer.

According to one embodiment of the invention, the organic electronic device comprises at least one photoactive

layer and the at least one of the at least one organic semiconductor layer is arranged between the anode and the at least one photoactive layer.

According to one embodiment of the invention, the organic electronic devices comprises at least one photoactive layer and the at least one organic semiconductor layers is arranged between the anode and the at least one photoactive layer.

According to one embodiment of the invention, the organic electronic device comprises at least two photoactive layers, wherein at least one organic semiconductor layers is arranged between the first and the second photoactive layer.

According to one embodiment of the invention, the organic electronic device comprises at least two photoactive layers, wherein at least one of the at least one organic semiconductor layers is arranged between the first and the second photoactive layer.

According to one embodiment of the invention, the organic electronic device comprises at least two photoactive layers, wherein one of the at least one organic semiconductor layers is arranged between the first and the second photoactive layer and one of the at least one organic semiconductor layers is arranged between the anode layer and the first photoactive layer.

According to one embodiment of the invention the electronic organic device is an electroluminescent device, preferably an organic light emitting diode.

The present invention furthermore relates to a display device comprising an organic electronic device according to the present invention.

## Further layers

In accordance with the invention, the organic electronic device may comprise, besides the layers already mentioned above, further layers. Exemplary embodiments of respective layers are described in the following:

### *Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### *Anode layer*

The anode layer may be formed by depositing or sputtering a material that is used to form the anode layer. The material used to form the anode layer may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode layer may also be formed using metals, typically silver (Ag), gold (Au), or metal alloys.

According to a preferred embodiment the organic electrode device comprises an anode layer, whereby the anode layer comprises a first anode sub-layer and a second anode sub-layer, wherein

- the first anode sub-layer comprises a first metal having a work function in the range of $\geq$ 4 and $\leq$ 6 eV, and
- the second anode sub-layer comprises a transparent conductive oxide; and
- the second anode sub-layer is arranged closer to the hole injection layer.

According to one embodiment of the present invention, the first metal of the first anode sub-layer may be selected from the group comprising Ag, Mg, Al, Cr, Pt, Au, Pd, Ni, Nd, Ir, preferably Ag, Au or Al, and more preferred Ag.

According to one embodiment of the present invention, the first anode sub-layer has have a thickness in the range of 5 to 200 nm, alternatively 8 to 180 nm, alternatively 8 to 150 nm, alternatively 100 to 150 nm.

According to one embodiment of the present invention, the first anode sub-layer is formed by depositing the first metal via vacuum thermal evaporation.

It is to be understood that the first anode layer is not part of the substrate.

According to one embodiment of the present invention, the transparent conductive oxide of the second anode sub layer is selected from the group selected from the group comprising indium tin oxide or indium zinc oxide, more preferred indium tin oxide.

According to one embodiment of the present invention, the second anode sub-layer may has a thickness in

the range of 3 to 200 nm, alternatively 3 to 180 nm, alternatively 3 to 150 nm, alternatively 3 to 20 nm.

According to one embodiment of the present invention, the second anode sub-layer may be formed by sputtering of the transparent conductive oxide.

According to one embodiment of the present invention, anode layer of the organic electronic device comprises in addition a third anode sub-layer comprising a transparent conductive oxide, wherein the third anode sub-layer is arranged between the substrate and the first anode sub-layer.

According to one embodiment of the present invention, the third anode sub-layer comprises a transparent oxide, preferably from the group selected from the group comprising indium tin oxide or indium zinc oxide, more preferred indium tin oxide.

According to one embodiment of the present invention, the third anode sub-layer may has a thickness in the range of 3 to 200 nm, alternatively 3 to 180 nm, alternatively 3 to 150 nm, alternatively 3 to 20 nm.

According to one embodiment of the present invention, the third anode sub-layer may be formed by sputtering of the transparent conductive oxide.

It is to be understood that the third anode layer is not part of the substrate.

According to one embodiment of the present invention, the hole injection layer is in direct contact with the anode layer.

*Hole injection layer*

A hole injection layer (HIL) may be formed on the anode layer by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of $10^{-8}$ to $10^{-3}$ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; $\alpha$-NPD (N,N'-Bis(naphthalen-1-yl)-N,N'-bis(phenyl)-benzidine) doped with F4TCNQ. $\alpha$-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to one embodiment of the present invention, the hole transport layer may comprise the same substantially covalent matrix compound as the organic semiconductor layer of the present invention.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Photoactive layer (PAL)*

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

According to one embodiment of the present invention, the photoactive layer does not comprise the compound of formula (I).

The photoactive layer may be a light-emitting layer or a light-absorbing layer preferably a light-emitting layer.

*Emission layer (EML)*

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML

According to one embodiment of the present invention, the emission layer does not comprise the compound of formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, $Ir(piq)_3$, and $Btp_2Ir(acac)$, but are not limited thereto. These compounds are phosphorescent emitters, however, fluorescent red emitter dopants could also be used.

Examples of phosphorescent green emitter dopants are $Ir(ppy)_3$ (ppy = phenylpyridine), $Ir(ppy)_2(acac)$, $Ir(mpyp)_3$.

Examples of phosphorescent blue emitter dopants are F2Irpic, $(F2ppy)_2Ir(tmd)$ and $Ir(dfppz)_3$ and ter-fluorene.

4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

*Hole blocking layer (HBL)*

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and azine derivatives, preferably triazine or pyrimidine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

*Electron transport layer (ETL)*

The organic electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

*Electron injection layer (EIL)*

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

*Cathode layer*

The cathode layer is formed on the ETL or optional EIL. The cathode layer may be formed of a metal, an alloy, an electrically conductive compound, or a mixture thereof. The cathode electrode may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer or the electron transport layer.

*Semiconductor layer*

According to one embodiment the semiconductor layer comprises at least one compound of Formula (I) and at least one substantially covalent matrix.

According to one embodiment the semiconductor layer comprises at least one compound of Formula (I) is a hole injection layer.

*Charge generation layer*

The term "n-type charge generation layer" is sometimes in the art also named n-CGL or electron generation layer and is intended to include the both.

The term "p-type charge generation layer" is sometimes in the art also named p-CGL or hole generation layer and is intended to include the both.

According to one embodiment of the present invention the p-type and/or n-type charge generation layer and/or the compound of formula (I) are non-emissive.

According to one embodiment of the invention, the p-type charge generation layer is arranged closer to the cathode layer than the n-type charge generation layer.

According to one embodiment of the invention, the p-type charge generation layer further comprises a substantially covalent matrix compound.

*Organic light-emitting diode (OLED)*

The organic electronic device according to the invention may be an organic light-emitting device.

According to one aspect of the present invention, there is provided an organic light-emitting diode (OLED) comprising: a substrate; an anode electrode formed on the substrate; an organic semiconductor layer comprising a compound of formula (I), a hole transport layer, an emission layer, an electron transport layer and a cathode electrode.

According to another aspect of the present invention, there is provided an OLED comprising: a substrate; an anode electrode formed on the substrate; an organic semiconductor layer comprising a compound of formula (I), a hole transport layer, an electron blocking layer, an emission layer, a hole blocking layer, an electron transport layer and a cathode electrode.

According to another aspect of the present invention, there is provided an OLED comprising: a substrate; an anode electrode formed on the substrate; an organic semiconductor layer comprising a compound of formula (I), a hole transport layer, an electron blocking layer, an emission layer, a hole blocking layer, an electron transport layer, an electron injection layer, and a cathode electrode.

According to another aspect of the present invention, there is provided an organic light-emitting diode (OLED) comprising: a substrate; an anode layer formed on the substrate; a charge generation layer according to invention, at least one emission layer and a cathode layer.

According to one aspect of the present invention, there is provided an organic light-emitting diode (OLED) comprising: a substrate; an anode layer formed on the substrate; a charge generation layer according to invention, at least a first and a second emission layers and a cathode layer, wherein the charge generation layer is arranged between the first and the second emission layer.

According to one aspect of the present invention, there is provided an organic light-emitting diode (OLED) comprising: a substrate; an anode layer formed on the substrate; a n-type charge generation layer, a p-type charge generation layer comprising a compound of formula (I),, a hole transport layer, an emission layer, an electron transport layer and a cathode layer.

According to another aspect of the present invention, there is provided an OLED comprising: a substrate; an anode layer formed on the substrate; a n-type charge generation layer, a p-type charge generation layer comprising a compound of formula (I), a hole transport layer, an electron blocking layer, an emission layer, a hole blocking layer, an electron transport layer and a cathode layer.

According to another aspect of the present invention, there is provided an OLED comprising: a substrate; an anode electrode formed on the substrate; a n-type charge generation layer, a p-type charge generation layer comprising a compound of formula (I), a hole transport layer, an electron blocking layer, an emission layer, a hole blocking layer, an electron transport layer, an electron injection layer, and a cathode electrode.

According to various embodiments of the present invention, there may be provided OLEDs layers arranged between the above mentioned layers, on the substrate or on the top electrode.

According to one aspect, the OLED may comprise a layer structure of a substrate that is adjacent arranged to

an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

According to one embodiment of the invention, at least one semiconductor layer is arranged and/or provided adjacent to the anode layer.

According to one embodiment of the invention, at least one semiconductor layer of the present invention is a hole-injection layer.

According to another aspect, the at least one semiconductor layer may have a layer thickness of at least about $\geq 0.5$ nm to about $\leq 10$ nm, preferably of about $\geq 2$ nm to about $\leq 8$ nm, also preferred of about $\geq 3$ nm to about $\leq 5$ nm.

The organic semiconductor layer according to the invention may be the first hole injection layer and/or the p-type charge generation layer.

For example, the OLED according to Fig. 2 may be formed by a process, wherein on a substrate (110), an anode layer (120), a hole injection layer (130), a hole transport layer (140), an electron blocking layer (145), an emission layer (150), a hole blocking layer (155), an electron transport layer (160), an electron injection layer (180) and the cathode layer (190) are subsequently formed in that order.

Organic electronic device

The organic electronic device according to the invention may be a light emitting device, thin film transistor, a battery, a display device or a photovoltaic cell, preferably a light emitting device or a photovoltaic cell, and most preferably a light emitting device.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

- a first deposition source to release the compound of formula (I) according to the invention, and
- a second deposition source to release the substantially covalent matrix compound;

the method comprising the steps of forming the organic semiconductor layer; whereby for an organic light-emitting diode (OLED):

- the organic semiconductor layer is formed by releasing the compound of formula (I) according to the invention from the first deposition source and the substantially covalent matrix compound from the second deposition source.

According to various embodiments of the present invention, the method may further include forming on the anode electrode, at least one layer selected from the group consisting of forming a hole transport layer or forming a hole blocking layer, and an emission layer between the anode electrode and the first electron transport layer.

According to various embodiments of the present invention, the method may further include the steps for forming an organic light-emitting diode (OLED), wherein

- on a substrate an anode electrode is formed,
- on the anode electrode an organic semiconductor layer comprising a compound of formula (I) is formed,

- on the organic semiconductor layer comprising a compound of formula (I) a hole transport layer is formed,
- on the hole transport layer an emission layer is formed,
- on the emission layer an electron transport layer is formed, optionally a hole blocking layer is formed on the emission layer,
- and finally a cathode electrode is formed,
- optional a hole blocking layer is formed in that order between the first anode electrode and the emission layer,
- optional an electron injection layer is formed between the electron transport layer and the cathode electrode.

According to various embodiments of the present invention, there is provided a method using:

- a first deposition source to release the compound of formula (I) according to the invention, and
- a second deposition source to release the substantially covalent matrix compound;
- a third deposition source to release the n-CGL matrix compound;
- a fourth deposition source to release the n-CGL dopant;

the method comprising the steps of forming the p-type charge generation layer; whereby for an organic light-emitting diode (OLED):

- the p-type charge generation layer is formed by releasing the compound of formula (I) according to the invention from the first deposition source and the substantially covalent matrix compound from the second deposition source;

the method comprising the steps of forming the n-type charge generation layer; whereby for an organic light-emitting diode (OLED):

- the n-type charge generation layer is formed by releasing the n-CGL matrix compound according to the invention from the third deposition source and n-CGL dopant from the fourth deposition source.

According to various embodiments, the OLED may have the following layer structure, wherein the layers having the following order:
anode, organic semiconductor layer comprising a compound of formula (I) according to the invention, first hole transport layer, second hole transport layer, emission layer, optional hole blocking layer, electron transport layer, optional electron injection layer, and cathode.

According to various embodiments of the present invention, the method may further include forming on the anode electrode, at least one layer selected from the group consisting of forming a hole transport layer or forming a hole blocking layer, an emission layer and a n-type charge generation layer between the anode electrode and the cathode layer.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

**Description of the Drawings**

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

FIG. 1 is a schematic sectional view of an organic electronic device, according to an exemplary embodiment of the present invention;

FIG. 2 is a schematic sectional view of an organic light-emitting diode (OLED), according to an exemplary embodiment of the present invention;

FIG. 3 is a schematic sectional view of an OLED, according to an exemplary embodiment of the present invention.

FIG. 4 is a schematic sectional view of a tandem OLED comprising a charge generation layer, according to an exemplary embodiment of the present invention.

FIG. 5 is a schematic sectional view of a tandem OLED comprising a charge generation layer, according to an exemplary embodiment of the present invention.

FIG. 6 is a schematic sectional view of an organic light-emitting diode (OLED), according to an exemplary embodiment of the present invention.

FIG. 7 is a schematic sectional view of an organic light-emitting diode (OLED), according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 100, according to an exemplary embodiment of the present invention. The organic electronic device 100 includes a substrate 110, an anode layer 120 and a hole injection layer (HIL) (130) which may comprise a compound of formula (I). The HIL 130 is disposed on the anode layer 120. Onto the HIL 130, a photoactive layer (PAL) 150 which may be light-emitting layer and a cathode layer 190 are disposed.

FIG. 2 is a schematic sectional view of an organic light-emitting diode (OLED) 100, according to an exemplary embodiment of the present invention. The OLED 100 includes a substrate 110, an anode layer 120 and a hole injection layer (HIL) 130 which may comprise a compound of formula (I). The HIL 130 is disposed on the anode layer 120. Onto the HIL 130, a hole transport layer (HTL) 140, an emission layer (EML) 150, an electron transport layer (ETL) 160, an electron injection layer (EIL) 180 and a cathode layer 190 are disposed. Instead of a single electron transport layer 160, optionally an electron transport layer stack (ETL) can be used.

Fig. 3 is a schematic sectional view of an OLED 100, according to another exemplary embodiment of the present invention. Fig. 3 differs from Fig. 2 in that the OLED 100 of Fig. 3 comprises an electron blocking layer (EBL) 145 and a hole blocking layer (HBL) 155.

Referring to Fig. 3, the OLED 100 includes a substrate 110, an anode layer 120, a hole injection layer (HIL) 130 which may comprise a compound of formula (I), a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode layer 190.

Fig. 4 is a schematic sectional view of an OLED 200, according to another exemplary embodiment of the present invention. Fig. 4 differs from Fig. 2 and 3 in that the OLED 200 of Fig. 4 comprises a charge generation layer (CGL) and an additional electron transport layer (ETL) 161 which replace the hole injection layer (HIL) 130 of OLED 100 in Fig. 3. Referring to Fig. 4 the OLED 200 includes a substrate 110, an anode 120, a first electron transport layer (ETL1) 161, an n-type charge generation layer (n-type CGL) 185, a p-type charge generation layer (p-type GCL) 135 which may comprise compound of Formula (I), a first hole transport layer (HTL) 140, a first electron blocking layer (EBL) 145, a first emission layer (EML) 150, a first hole blocking layer (HBL) 155, a second electron transport layer (ETL2) 160, and a cathode 190.

Fig. 5 shows the OLED 200 including a substrate 110, an anode layer 120, a hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-CGL) 185, a p-type charge generation layer (p-GCL) 135 which may comprise compound of Formula (I), a second hole transport layer (HTL) 141, a second electron blocking layer (EBL) 146, a second emission layer (EML) 151, a second hole blocking layer (EBL) 156, a second electron transport layer (ETL) 161, an electron injection layer (EIL) 180 and a cathode layer 190. The HIL may comprise a compound of Formula (I).

FIG. 6 is a schematic sectional view of an organic light-emitting diode (OLED) 100, according to an exemplary embodiment of the present invention. The OLED 100 includes a substrate (110), an anode layer (120) that comprises a first anode sub-layer (121) and a second anode sub-layer (122), a semiconductor layer comprising compound of Formula (I) (130), a hole transport layer (HTL) (140), an electron blocking layer (EBL) (145), an emission layer (EML) (150), a hole blocking layer (EBL) (155), an electron transport layer (ETL) (160) and a cathode layer (190).

FIG. 7 is a schematic sectional view of an organic light-emitting diode (OLED) 100, according to an exemplary embodiment of the present invention. The OLED 100 includes a substrate (110), an anode layer (120) that comprises

a first anode sub-layer (121), a second anode sub-layer (122) and a third anode sub-layer (123), a semiconductor layer comprising compound of Formula (I) (130), a hole transport layer (HTL) (140), an electron blocking layer (EBL) (145), an emission layer (EML) (150), a hole blocking layer (EBL) (155), an electron transport layer (ETL) (160) and a cathode layer (190). The layers are disposed exactly in the order as mentioned before.

While not shown in Fig. 1 to Fig. 7, a sealing layer may further be formed on the cathode layer 190, in order to seal the organic electronic device 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

**Detailed description**

The invention is furthermore illustrated by the following examples which are illustrative only and non-binding.

Compounds of formula (I) may be prepared as described in EP2180029A1 and WO2016097017A1.

Thermogravimetric analysis

The term "TGA5%" denotes the temperature at which 5 % weight loss occurs during thermogravimetric analysis and is measured in °C.

The TGA5% value may be determined by heating a 9-11 mg sample in a thermogravimetric analyzer at a heating rate of 10 K/min in an open 100 μL aluminum pan, under a stream of nitrogen at a flow rate of 20 mL/min in the balance area and of 30 mL/min in the oven area.

The TGA5% value may provide an indirect measure of the volatility and/or decomposition temperature of a compound. In first approximation, the higher the TGA5% value the lower is the volatility of a compound and/or the higher the decomposition temperature.

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

General procedure for fabrication of OLEDs

For the examples according to the invention and comparative examples in Table 3, a glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The liquid film was removed in a nitrogen stream, followed by plasma treatment, see Table 2, to prepare the anode layer. The plasma treatment was performed in an atmosphere comprising 97.6 vol.-% nitrogen and 2.4 vol.-% oxygen.

Then, N-({[1,1-'biphenyl]-4-yl)-9,9,dimethyl-N-(4-(9-phenyl-9H-carbazol-3-yl)phenyl)-9H-fluoren-2-amine} (compound F3, cf. above) and compound of formula (I) or a comparative compound according to Table 3 were vacuum deposited on the anode, to form a HIL having a thickness of 10 nm. The amount of compound of formula (I) in the HIL can be seen in Table 3.

Then, N-({[1,1-'biphenyl]-4-yl)-9,9,dimethyl-N-(4-(9-phenyl-9H-carbazol-3-yl)phenyl)-9H-fluoren-2-amine} was vacuum deposited on the HIL, to form a first HTL having a thickness of 121 nm.

Then N-([1,1'-biphenyl]-4-yl)-9,9-diphenyl-N-(4-triphenylsilyl)phenyl)-9H-fluoren-2-amine (CAS 1613079-70-1) was vacuum deposited on the HTL, to form an electron blocking layer (EBL) having a thickness of 5 nm.

Then 97 vol.-% H09 (Sun Fine Chemicals, Korea) as EML host and 3 vol.-% BD200 (Sun Fine Chemicals, Korea) as fluorescent blue dopant were deposited on the EBL, to form a first blue-emitting EML with a thickness of 20 nm.

Then the hole blocking layer is formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer.

Then, the electron transporting layer (ETL) having a thickness of 31 nm is formed on the hole blocking layer by depositing 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% LiQ.

Then Yb was evaporated at a rate of 0.01 to 1 Å/s at $10^{-7}$ mbar to form an electron injection layer with a thickness of 2nm on the electron transporting layer.

Ag/Mg (90:10 vol%) is evaporated at a rate of 0.01 to 1 Å/s at $10^{-7}$ mbar to form a cathode with a thickness of 13 nm.

Then, N-({[1,1-'biphenyl]-4-yl)-9,9,dimethyl-N-(4-(9-phenyl-9H-carbazol-3-yl)phenyl)-9H-fluoren-2-amine} was vacuum deposited on the cathode layer to form a capping layer with a thickness of 75 nm.

The OLED stack is protected from ambient conditions by encapsulation of the device with a glass slide. Thereby, a cavity is formed, which includes a getter material for further protection.

Organic electronic device comprising a charge generation layer (CGL)

For the top emission OLED devices, a substrate with dimensions of 150 mm x 150 mm x 0.7 mm was ultrasonically cleaned with 2% aqueous solution of Deconex FPD 211 for 7 minutes and then pure water for 5 minutes, and dried for 15 minutes in a spin dryer. Subsequently, Ag was deposited as anode at a pressure of $10^{-5}$ to $10^{-7}$ mbar on the substrate.

Then N-([1,1'-biphenyl]-2-yl)-N-(9.9-dimethyl-9H-fluoren-2-yl)-9,9'spirobi[fluoren]-2-amine was vacuum deposited with 8 vol% 4,4',4"-((1E,1'E,1"E)-cyclopropane-1,2,3-triylidenetris(cyanomethanylylidene))tris(2,3,5,6-tetrafluoobenzonitrile) to form a hole injection layer having a thickness 10 nm.

Then N-([1,1'-biphenyl]-2-yl)-N-(9.9-dimethyl-9H-fluoren-2-yl)-9,9'spirobi[fluoren]-2-amine was vacuum deposited, to form a first hole transport layer having a thickness of 24 nm

Then N-([1,1'-biphenyl]-4-yl)-9,9-diphenyl-N-(4-triphenylsilyl)phenyl)-9H-fluoren-2-amine was vacuum deposited on the HTL, to form an electron blocking layer (EBL) having a thickness of 5 nm.

Then 97 vol.-% H09 (Sun Fine Chemicals, Korea) as EML host and 3 vol.-% BD200 (Sun Fine Chemicals, Korea) as fluorescent blue dopant were deposited on the EBL, to form a first blue-emitting EML with a thickness of 20 nm.

Then 2-(3'-(9,9-dimethyl-9H-fluoren-2-yl)-[1,1'-biphenyl]-3-yl)-4,6-diphenyl-1,3,5-triazine was vacuum deposited to form a first hole blocking layer having a thickness of 5 nm.

Then, 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% LiQ were vacuum deposited on the first hole blocking layer to form a first electron transport layer having a thickness of 25nm.

Then the first n-type CGL having a thickness of 15 nm is formed on the ETL1 by co-depositing 99 vol.-% 2,2'-(1,3-Phenylene)bis[9-phenyl-1,10-phenanthroline] and 1 vol.-% Li.

Then the first p-type CGL having a thickness of 10 nm is formed on the first n-type CGL by co-depositing N-([1,1'-biphenyl]-2-yl)-N-(9.9-dimethyl-9H-fluoren-2-yl)-9,9'spirobi[fluoren]-2-amine with a compound according to formula (I) or a comparative example according to table 4.

Then a second hole transport layer having a thickness of 36 nm is formed on the first p-type CGL by depositing N-([1,1'-biphenyl]-2-yl)-N-(9.9-dimethyl-9H-fluoren-2-yl)-9,9'spirobi[fluoren]-2-amine.

Then a second electron blocking layer having a thickness of 5 nm is formed on the second hole transport layer by depositing N-([1,1'-biphenyl]-4-yl)-9,9-diphenyl-N-(4-triphenylsilyl)phenyl)-9H-fluoren-2-amine,

Then 97 vol.-% H09 (Sun Fine Chemicals, Korea) as EML host and 3 vol.-% BD200 (Sun Fine Chemicals, Korea) as fluorescent blue dopant were deposited on the second EBL, to form a second blue-emitting EML with a thickness of 20 nm.

Then a second hole blocking layer having a thickness of 5 nm is formed on the second blue-emitting EML

Then, 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% LiQ were vacuum deposited on the second hole blocking layer to form a second electron transport layer having a thickness of 25nm.

Then the second n-type CGL having a thickness of 15 nm is formed on the ETL2 by co-depositing 99 vol.-% 2,2'-(1,3-Phenylene)bis[9-phenyl-1,10-phenanthroline] and 1 vol.-% Li.

Then the second p-type CGL having a thickness of 10 nm is formed on the second n-type CGL by co-depositing N-([1,1'-biphenyl]-2-yl)-N-(9.9-dimethyl-9H-fluoren-2-yl)-9,9'spirobi[fluoren]-2-amine with a compound according to formula (I) or a comparative example according to Table 4.

Then a third hole transport layer having a thickness of 57 nm is formed on the second p-type CGL by depositing N-([1,1'-biphenyl]-2-yl)-N-(9.9-dimethyl-9H-fluoren-2-yl)-9,9' spirobi[fluoren]-2-amine.

Then a third electron blocking layer having a thickness of 5 nm is formed on the third hole transport layer by depositing N-([1,1'-biphenyl]-4-yl)-9,9-diphenyl-N-(4-triphenylsilyl)phenyl)-9H-fluoren-2-amine,

Then 97 vol.-% H09 (Sun Fine Chemicals, Korea) as EML host and 3 vol.-% BD200 (Sun Fine Chemicals, Korea) as fluorescent blue dopant were deposited on the third EBL, to form a third blue-emitting EML with a thickness

of 20 nm.

Then a third hole blocking layer having a thickness of 5 nm is formed on the third blue-emitting EML

Then, 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% LiQ were vacuum deposited on the third hole blocking layer to form a third electron transport layer having a thickness of 31 nm.

Then Yb was evaporated at a rate of 0.01 to 1 Å/s at $10^{-7}$ mbar to form an electron injection layer with a thickness of 2nm on the electron transporting layer.

Ag/Mg (90:10 vol%) is evaporated at a rate of 0.01 to 1 Å/s at $10^{-7}$ mbar to form a cathode with a thickness of 13 nm.

Then, N-({[1,1-'biphenyl]-4-yl)-9,9,dimethyl-N-(4-(9-phenyl-9H-carbazol-3-yl)phenyl)-9H-fluoren-2-amine} was vacuum deposited on the cathode layer to form a capping layer with a thickness of 75 nm.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in $cd/m^2$ using an Instrument Systems CAS-140CT array spectrometer (calibrated by Deutsche Akkreditierungsstelle (DAkkS)) for each of the voltage values. The cd/A efficiency at 10 mA/cm2 is determined by interpolating the luminance-voltage and current-voltage characteristics, respectively.

In bottom emission devices, the emission is predominately Lambertian and quantified in percent external quantum efficiency (EQE). To determine the efficiency EQE in % the light output of the device is measured using a calibrated photodiode at 10 mA/cm2.

In top emission devices, the emission is forward directed, non-Lambertian and also highly dependent on the mirco-cavity. Therefore, the efficiency EQE will be higher compared to bottom emission devices. To determine the efficiency EQE in % the light output of the device is measured using a calibrated photodiode at 10 $mA/cm^2$.

Lifetime LT of the device is measured at ambient conditions (20°C) and 30 $mA/cm^2$, using a Keithley 2400 sourcemeter, and recorded in hours.

The brightness of the device is measured using a calibrated photo diode. The lifetime LT is defined as the time till the brightness of the device is reduced to 97 % of its initial value.

The increase in operating voltage ΔU is used as a measure of the operational voltage stability of the device. This increase is determined during the LT measurement and by subtracting the operating voltage after 1 hour after the start of operation of the device from the operating voltage after 100 hours.

$$\Delta U=[U100\ h)-\ U(1h)]$$

or the operating voltage after 1 hour after the start of operation of the device from the operating voltage after 400 hours.

$$\Delta U=[U400\ h)-\ U(1h)]$$

The smaller the value of ΔU the better is the operating voltage stability.

***Technical Effect of the invention***

In Table 1 and 2 are shown LUMO levels for inventive compounds C1 to C25. LUMO levels were calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase.

Table 1: Calculated LUMO levels of compounds of formula (I), whereby R', R" and R''' are CN and $Ar^1$, $Ar^2$ and $Ar^3$ as shown.

| Compound | $Ar^1$ | $Ar^2$ | $Ar^3$ | LUMO [eV] |
|---|---|---|---|---|
| E1 | 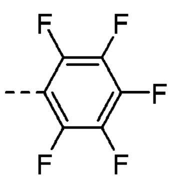 | 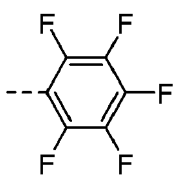 | 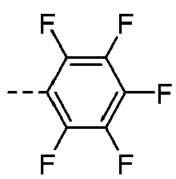 | -4.58 |

(continued)

| Compound | Ar[1] | Ar[2] | Ar[3] | LUMO [eV] |
|---|---|---|---|---|
| E2 | 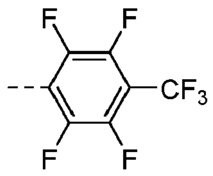 | 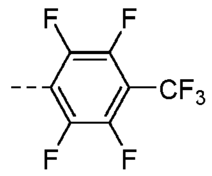 | 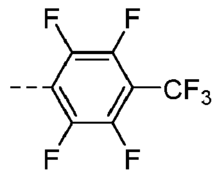 | -4.90 |
| E3 | 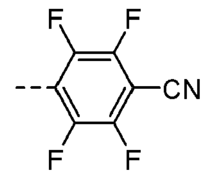 | 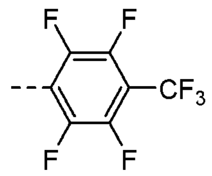 | 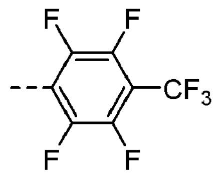 | -5.00 |
| C1 | 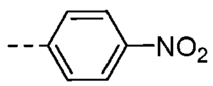 | 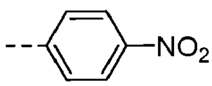 | 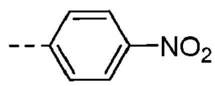 | -4.86 |
| C2 | 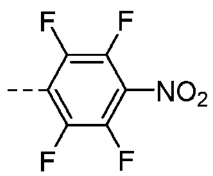 | 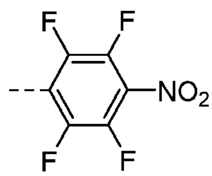 | 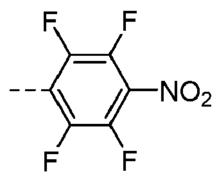 | -5.21 |
| C3 | 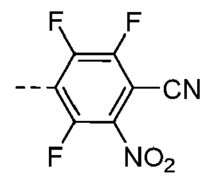 | 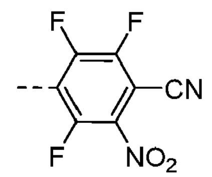 | 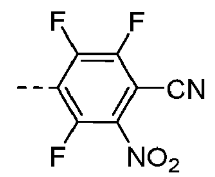 | -5.44 |
| C4 | 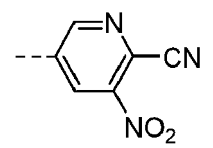 | 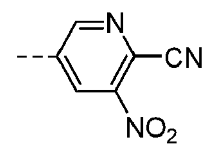 | 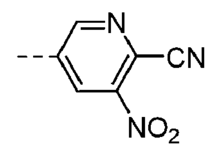 | -5.64 |
| C5 | 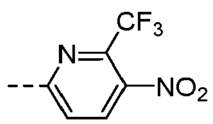 | 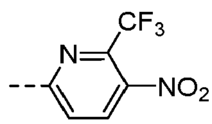 | 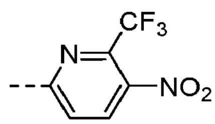 | -5.29 |
| C6 | 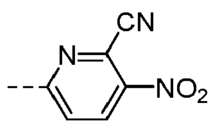 | 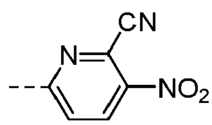 | 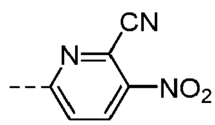 | -5.55 |
| C7 | 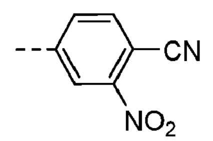 | 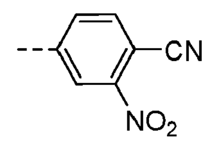 | 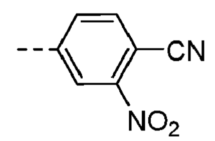 | -5.29 |
| C8 | 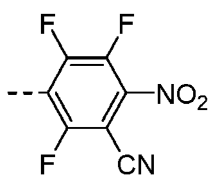 | 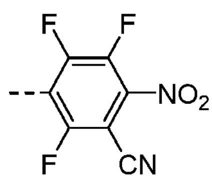 | 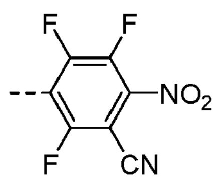 | -5.45 |

(continued)

| Compound | $Ar^1$ | $Ar^2$ | $Ar^3$ | LUMO [eV] |
|---|---|---|---|---|
| C9 | 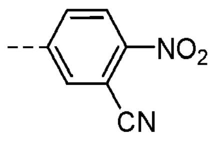 | 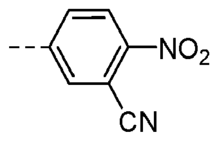 | 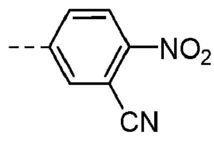 | -5.43 |
| C10 | 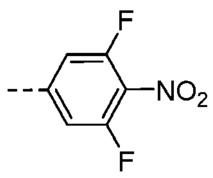 | 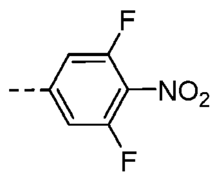 | 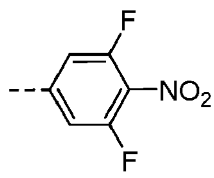 | -4.89 |
| C11 | 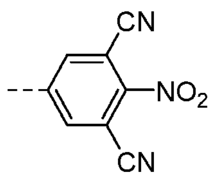 | 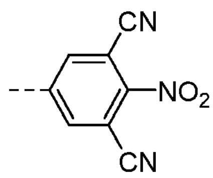 | 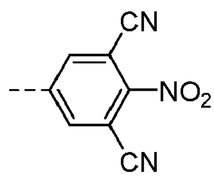 | -5.87 |
| C12 | 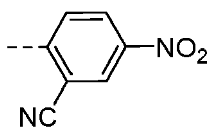 | 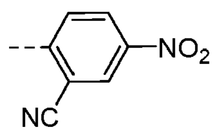 | 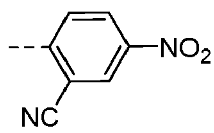 | -5.19 |
| C13 | 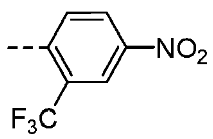 | 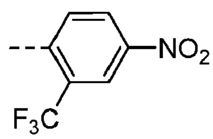 | 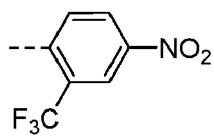 | -4.88 |
| C14 | 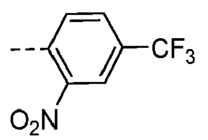 | 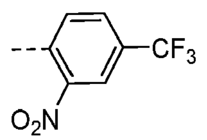 | 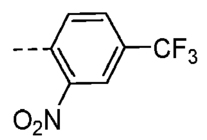 | -4.48 |
| C15 | 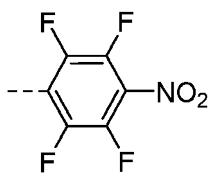 | 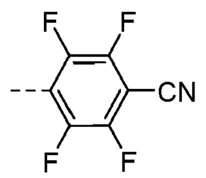 | 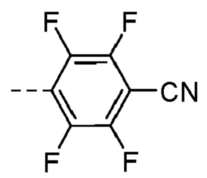 | -5.21 |
| C16 | 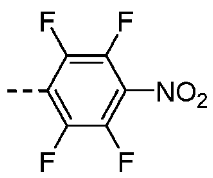 | 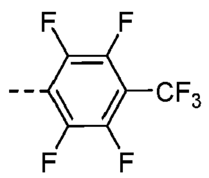 | 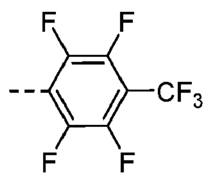 | -5.05 |
| C17 | 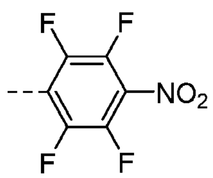 | 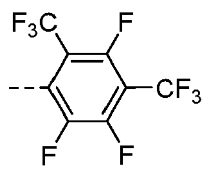 | 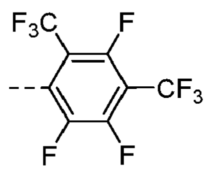 | -4.99 |

(continued)

| Compound | $Ar^1$ | $Ar^2$ | $Ar^3$ | LUMO [eV] |
|---|---|---|---|---|
| C18 | 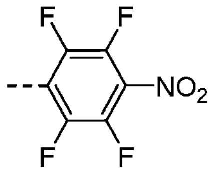 | 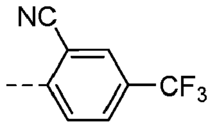 | 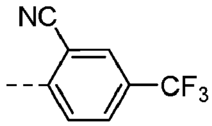 | -4.92 |
| C19 | 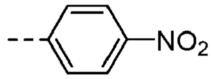 | 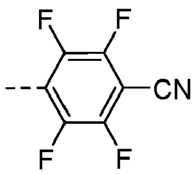 | 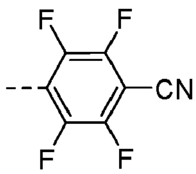 | -5.09 |
| C20 | 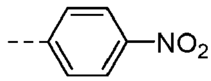 | 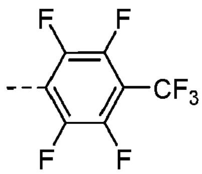 | 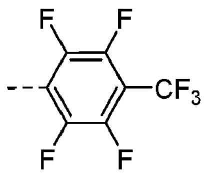 | -4.91 |
| C21 | 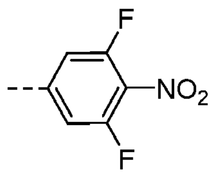 | 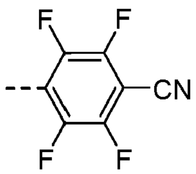 | 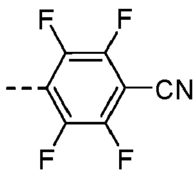 | -5.1 |
| C22 | 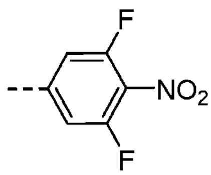 | 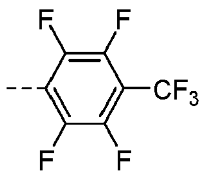 | 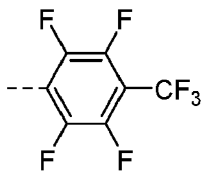 | -5.01 |
| C23 | 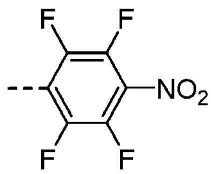 | 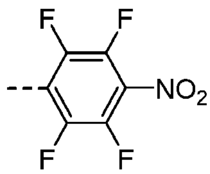 | 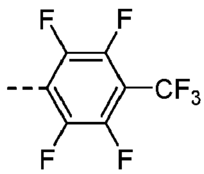 | -5.18 |
| C24 | 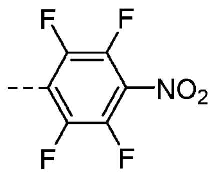 | 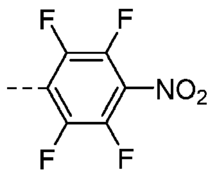 | 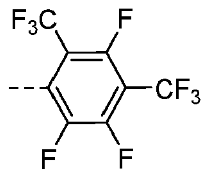 | -5.12 |
| C25 | 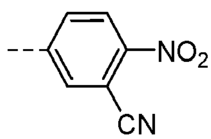 | 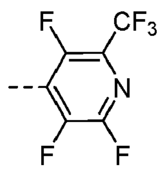 | 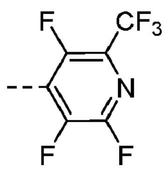 | -5.36 |

As comparative compounds the compounds E1 to E3 as shown in table 1 were used

In Table 2 the TGA 5% [°C] and $T_g$ [°C] for three inventive compounds and the three comparative compounds are shown.

Table 2: TGA 5% [°C] and $T_g$ [°C] for three inventive compounds and the three comparative compounds

| Material | TGA 5% [°C] | $T_g$ [°C] |
|---|---|---|
| E1 | *264* | *65* |
| E2 | *270* | *78* |
| E3 | *295* | *89* |
| C15 | *339* | *118* |
| C16 | *295* | 91 |
| C20 | 322 | 93 |

As can be seen from table 2, the TGA5% value may be higher than for the comparative compounds. The glass transition temperature may be higher than for the comparative compounds.

A higher TGA5% may be an indication for a lower volatility which may be beneficial for the manufacturing of an electronic device.

A higher glass transition temperature (Tg) may be beneficial for a longer life time of an electronic device.

In Table 3 performance data for organic electroluminescent devices comprising a hole injection layer (HIL) comprising comparative and inventive compounds are shown:

Table 3: Organic electronic devices comprising an hole injection layer (HIL) comprising comparative and inventive compounds.

| Mater ial | C (vol%) | CIE-y | U [V] at 10 mA/cm$^2$ | Ceff ([10 mA/cm$^2$] [cd/A]) | LT97 at 30 mA/c m$^2$ [h] | ΔV (100-1h) [30mA/cm $^2$,RT] [V] |
|---|---|---|---|---|---|---|
| E1 | 5 | 0.048 | 4.08 | 6.34 | 39 | 0.15 |
| E2 | 3 | 0.046 | 3.73 | 6.27 | 29 | 0.06 |
| E3 | 4 | 0.044 | 3.75 | 6.46 | 72 | 0.07 |
| C15 | 3 | 0.048 | 3.74 | 6.52 | 92 | 0.02 |
| C16 | 5 | 0.041 | 3.66 | 5.90 | 108 | 0.02 |
| C = concentration; Ceff = current efficiency; LT = Life time; ΔV = voltage rise. | | | | | | |

As can be seen from Table 3, LT97 at 30 mA/cm$^2$ is higher than for the comparative examples

A long lifetime may be beneficial for long-time stability of devices.

In Table 4 performance data for organic electroluminescent devices comprising a p-type charge injection layer (p-type CGL) comprising inventive compounds are shown.

Table 4: Organic electronic devices comprising a p-type charge injection layer (p-type CGL) comprising inventive compounds

| Material | Concen tration (%) | Ceff ([15mA/cm$^2$ ] [cd/A]) | LT97 at 30 mA/cm$^2$ [h] | ΔV (400-1h) [30mA/cm$^2$, RT] [V] |
|---|---|---|---|---|
| E3 | 10 | 23.16 | 74 | 0.85 |
| C15 | 10 | 25.05 | 133 | 0.41 |
| C16 | 10 | 23.82 | 103 | 0.43 |

As can be seen from Table 4, the current efficiency may be higher than for the comparative example. LT97 at 30 mA/cm2 may be higher than for the comparative example. The voltage rise may be lower than for the comparative example.

A long lifetime may be beneficial for long-time stability of devices.

A high efficiency may be beneficial for reduced power consumption and improved battery life, in particular in mobile devices.

A reduced increase in operating voltage over time may be an indication for improved stability of the electronic device.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention’s scope is defined in the following claims and the equivalents thereto. Furthermore, reference signs used in the description and claims do not limit the scope of the invention as claimed.

## Claims

1. **1.** A compound of formula (I)

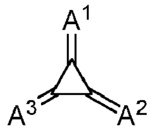

(I)

wherein $A^1$, $A^2$ and $A^3$ are represented by formula (II), (III), (IV), respectively

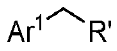 (II), 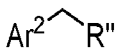 (III), and 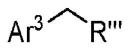 (IV)

whereby $Ar^1$, $Ar^2$ and $Ar^3$ are independently selected from substituted or unsubstituted aryl, substituted or unsubstituted $C_6$ to $C_{24}$ aryl, substituted or unsubstituted heteroaryl or $C_2$ to $C_{24}$ heteroaryl, wherein the substituents on $Ar^1$, $Ar^2$ and $Ar^3$ are independently selected form an electron-withdrawing group, $NO_2$, CN, halogen, Cl, F, partially fluorinated or perfluorinated alkyl and partially fluorinated or perfluorinated $C_1$ to $C_{12}$ alkyl, partially fluorinated or perfluorinated alkoxy, partially fluorinated or perfluorinated $C_1$ to $C_6$ alkoxy or D;
R’, R" and R’’’ are independently selected from substituted or unsubstituted aryl, substituted or unsubstituted $C_6$ to $C_{18}$ aryl, substituted or unsubstituted heteroaryl, substituted or unsubstituted $C_2$ to $C_{18}$ heteroaryl, electron-withdrawing group, partially fluorinated or perfluorinated alkyl, partially fluorinated or perfluorinated $C_1$ to $C_6$ alkyl, halogen, F, CN, and $NO_2$;
wherein the compound of formula (I) contains at least one $NO_2$ group;

or a salt of a radical anion of formula (I).

**2.** The compound of Claim 1, whereby $Ar^1$ is substituted with at least one $NO_2$ group

**3.** The compound of Claim 1 or 2, whereby $Ar^1$ is represented by formula (V)

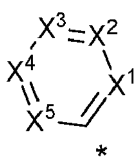

(V)

wherein $X^1$ is selected from $CR^1$ or N;
$X^2$ is selected from $CR^2$ or N;
$X^3$ is selected from $CR^3$ or N;

$X^4$ is selected from $CR^4$ or N;
$X^5$ is selected from $CR^5$ or N;
wherein one of $R^1$, $R^2$, $R^3$, $R^4$, and $R^5$ is $NO_2$ and the other $R^1$, $R^2$, $R^3$, $R^4$, and $R^5$ are independently selected from electron-withdrawing group, $NO_2$, CN, halogen, Cl, F, partially fluorinated or perfluorinated alkyl, partially fluorinated or perfluorinated $C_1$ to $C_{10}$ alkyl, partially fluorinated or perfluorinated alkoxy, partially fluorinated or perfluorinated $C_1$ to $C_6$ alkoxy, D or H;
wherein at least two of $X^1$ to $X^5$ are independently selected from $CR^1$ to $CR^5$; and wherein the asterix "*" denotes the binding position.

**4.** The compound of any of the claims 1 to 3, whereby $R^3$ is $NO_2$.

**5.** The compound of any of the claims 1 to 4, selected of the formula (VI)

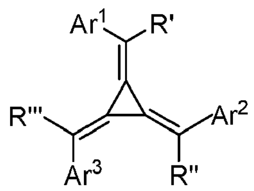

(VI)

**6.** The compound of any of the claims 1 to 5, whereby at least one $Ar^2$ and $Ar^3$ is substituted with at least one $NO_2$.

**7.** The compound of any of the claims 1 to 6, whereby at least one of $Ar^2$ and $Ar^3$ does not contain hydrogen.

**8.** The compound any of the claims 1 to 7, whereby $Ar^2$ and $Ar^3$ are identical.

**9.** The compound of any of the claims 1 to 8, whereby $Ar^2$ is represented by formula (VI) and $Ar^3$ is represented by formula (VII)

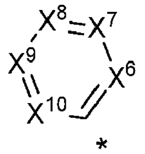

(VI)

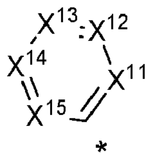

(VII)

wherein $X^6$ is selected from $CR^6$ or N;
$X^7$ is selected from $CR^7$ or N;
$X^8$ is selected from $CR^8$ or N;
$X^9$ is selected from $CR^9$ or N;
$X^{10}$ is selected from $CR^{10}$ or N;
wherein $X^{11}$ is selected from $CR^{11}$ or N;
$X^{12}$ is selected from $CR^{12}$ or N;
$X^{13}$ is selected from $CR^{13}$ or N;
$X^{14}$ is selected from $CR^{14}$ or N;
$X^{15}$ is selected from $CR^{15}$ or N;
wherein $R^6$, $R^7$, $R^8$, $R^9$, $R^{10}$, $R^{11}$, $R^{12}$, $R^{13}$, $R^{14}$, and $R^{15}$ are independently selected from electron-withdrawing group, $NO_2$, CN, halogen, Cl, F, partially fluorinated or perfluorinated alkyl, partially fluorinated or perfluorinated $C_1$ to $C_{10}$ alkyl, partially fluorinated or perfluorinated alkoxy, partially fluorinated or perfluorinated $C_1$ to $C_6$ alkoxy, D or H;

wherein at least two of $X^6$ to $X^{10}$ are independently selected from $CR^6$ to $CR^{10}$; wherein at least two of $X^{11}$ to $X^{15}$ are independently selected from $CR^{11}$ to $CR^{15}$; and wherein the asterix "*" denotes the binding position.

**10.** An organic semiconductor layer comprising at least one compound of formula (I) according to any of the preceding claims 1 to 9.

**11.** An organic electronic device comprising at least one organic semiconductor layer of claim 10.

**12.** The organic electronic device of claim 11, whereby the organic electronic device comprises an anode layer, a cathode layer, at least one photoactive layer and at least one organic semiconductor layer, wherein the at least one organic semiconductor layer is arranged between the anode layer and at least one photoactive layer

**13.** The organic electronic device of any of the claims 11 to 12, whereby the organic electronic device comprises at least two photoactive layers, wherein at least one organic semiconductor layer is arranged between the first and the second photoactive layer.

**14.** The organic electronic device of any of the claims 11 to 13, whereby the electronic organic device is a light emitting device, a thin film transistor, a battery, a display device or a photovoltaic cell, and preferably a light emitting device, preferably an organic light emitting diode.

**15.** A display device comprising an organic electronic device according to any of the claims 11 to 14.

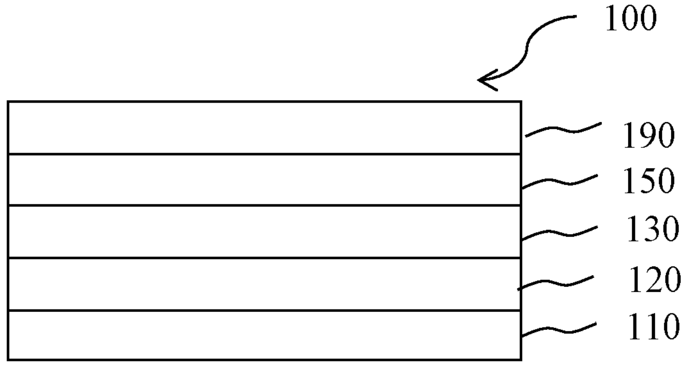


FIG. 1

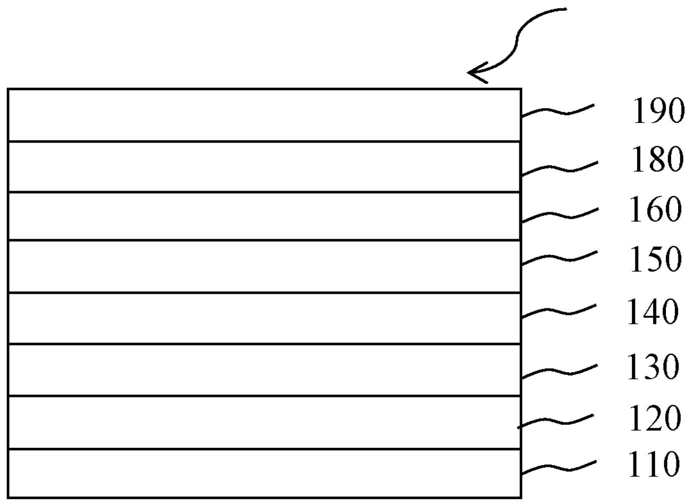


FIG. 2

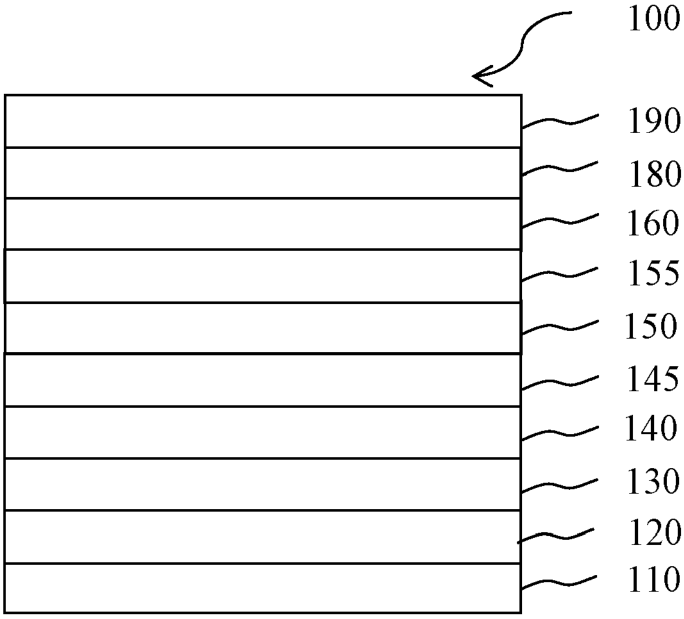


FIG. 3

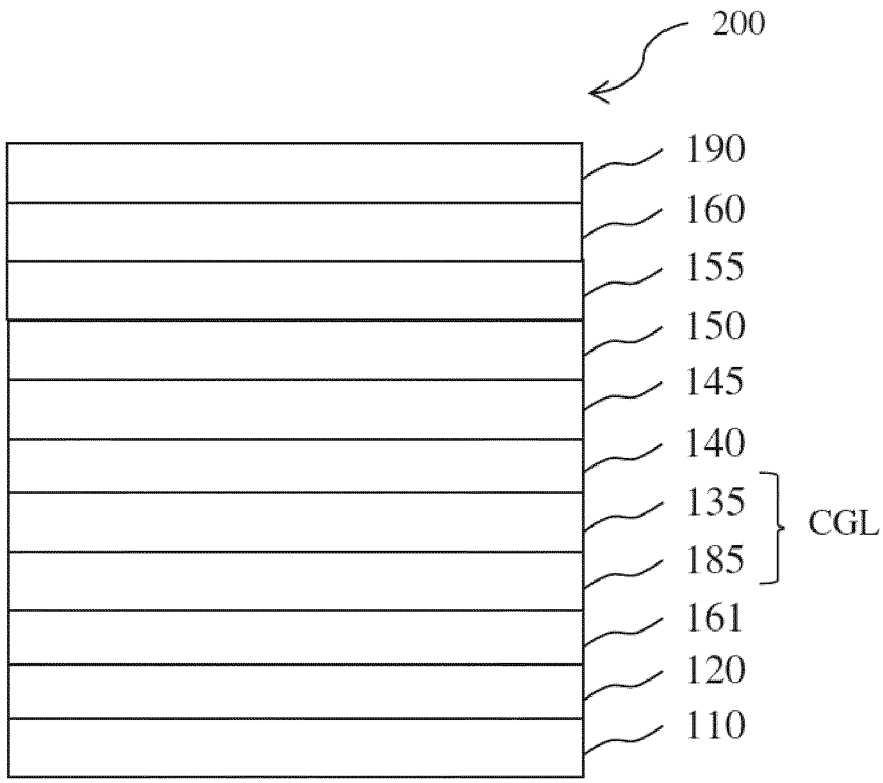


Fig. 4

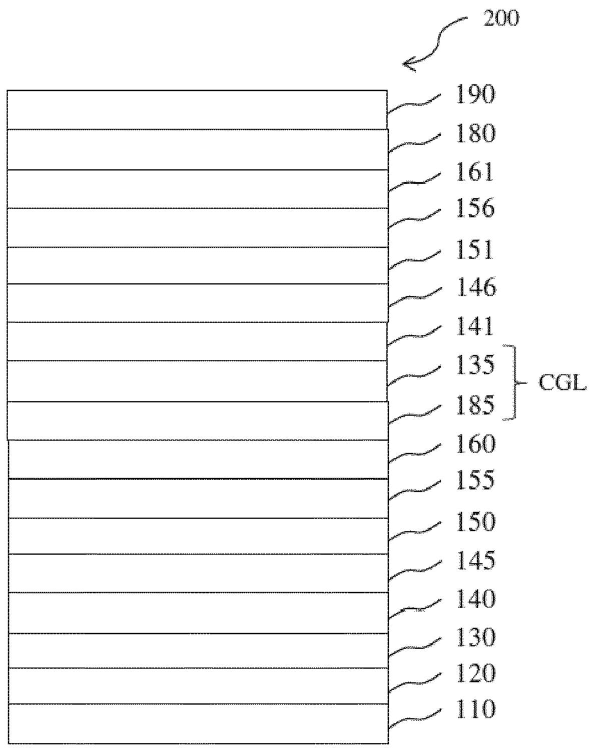
200
190
180
161
156
151
146
141
135
185
CGL
160
155
150
145
140
130
120
110

Fig. 5

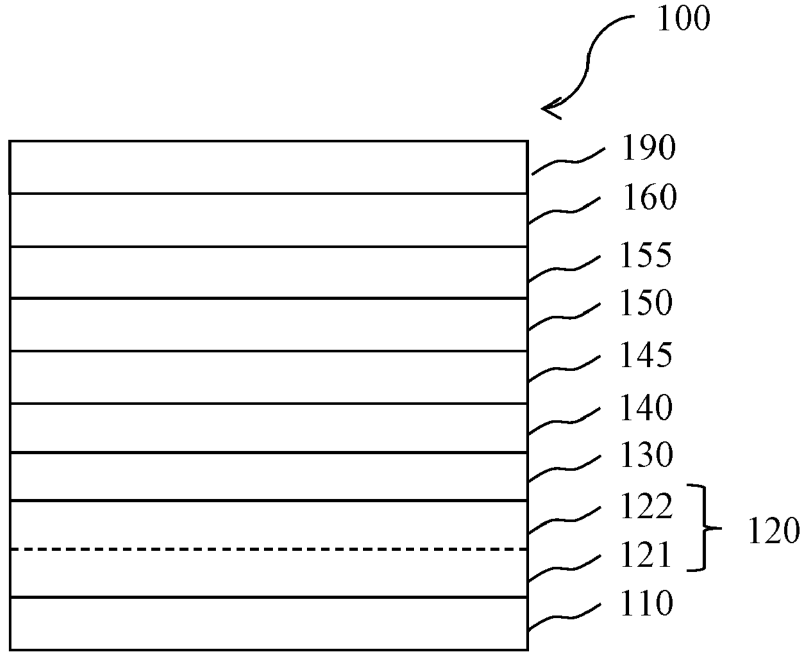


Fig. 6

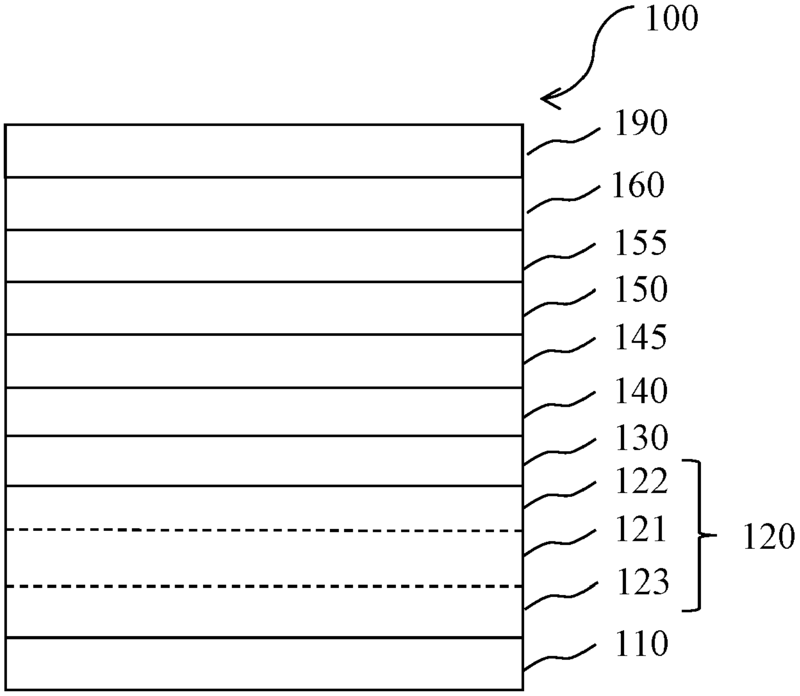


Fig. 7

Europäisches Patentamt
European Patent Office
Office européen des brevets

# EUROPEAN SEARCH REPORT

Application Number
EP 21 15 4618

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ENOMOTO TETSUYA ET AL: "Synthesis, Structure, and Properties of Hexaaryl[3]radialenes", BULLETIN OF THE CHEMICAL SOCIETY OF JAPAN, vol. 73, no. 9, 1 September 2000 (2000-09-01), pages 2109-2114, XP055822263, JP ISSN: 0009-2673, DOI: 10.1246/bcsj.73.2109 Retrieved from the Internet: URL:http://dx.doi.org/10.1246/bcsj.73.2109 > | 1-6,8,9 | INV. C07D213/61 C07D213/84 C07C255/35 C07C255/51 H01L51/00 |
| Y | * Chart I; page 2110; compound If * | 1-15 | |
| Y | CN 109 081 791 A (AAC TECH NANJING INC) 25 December 2018 (2018-12-25) * page 30 - page 33; claims; examples * | 1-15 | |
| Y | EP 3 034 489 A1 (NOVALED GMBH [DE]) 22 June 2016 (2016-06-22) * examples * | 1-15 | TECHNICAL FIELDS SEARCHED (IPC) |
| Y,D | EP 2 180 029 B1 (NOVALED AG [DE]) 27 July 2011 (2011-07-27) * examples * | 1-15 | C07D C07C H01L |

The present search report has been drawn up for all claims

2

| Place of search | Date of completion of the search | Examiner |
|---|---|---|
| Munich | 9 July 2021 | Österle, Carmen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 15 4618

This annex lists the patent family members relating to the patent documents cited in the above-mentioned European search report. The members are as contained in the European Patent Office EDP file on
The European Patent Office is in no way liable for these particulars which are merely given for the purpose of information.

09-07-2021

| Patent document cited in search report | Publication date | Patent family member(s) | Publication date |
|---|---|---|---|
| CN 109081791 A | 25-12-2018 | NONE | |
| EP 3034489 A1 | 22-06-2016 | CN 107207420 A | 26-09-2017 |
| | | EP 3034489 A1 | 22-06-2016 |
| | | EP 3233787 A1 | 25-10-2017 |
| | | JP 6649952 B2 | 19-02-2020 |
| | | JP 2018506137 A | 01-03-2018 |
| | | KR 20170097707 A | 28-08-2017 |
| | | TW 201638366 A | 01-11-2016 |
| | | US 2017373251 A1 | 28-12-2017 |
| | | WO 2016097017 A1 | 23-06-2016 |
| EP 2180029 B1 | 27-07-2011 | CN 101728485 A | 09-06-2010 |
| | | EP 2180029 A1 | 28-04-2010 |
| | | ES 2370120 T3 | 12-12-2011 |
| | | JP 5199978 B2 | 15-05-2013 |
| | | JP 2010100621 A | 06-05-2010 |
| | | KR 20100045402 A | 03-05-2010 |
| | | TW 201026648 A | 16-07-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2180029 A1
- EP 2722908 A1
- WO 2016097017 A1


### Non-patent literature cited in the description


- **YASUHIKO SHIROTA ; HIROSHI KAGEYAMA.** *Chem. Rev.,* 2007, vol. 107, 953-1010
- *CHEMICAL ABSTRACTS,* 1613079-70-1